(19) Europäisches Patentamt
European Patent Office
Office européen des brevets

(11) Publication number: **0 172 962 A1**

(12) **EUROPEAN PATENT APPLICATION**

(21) Application number: **84305959.3**

(22) Date of filing: **30.08.84**

(51) Int. Cl.⁴: **C 07 C 177/00**, C 08 B 37/16, A 61 K 31/557

(43) Date of publication of application: **05.03.86 Bulletin 86/10**

(84) Designated Contracting States: **AT BE CH DE FR GB IT LI LU NL SE**

(71) Applicant: **ONO PHARMACEUTICAL CO., LTD., No. 14, Doshomachi 2-chome Higashi-ku, Osaka-shi Osaka (JP)**

(72) Inventor: **Arai, Yoshinobu, 1-5-8-505, Wakayama-dai Shimamoto-cho, Mishima-gun Osaka (JP)**
Inventor: **Wakatsuka, Hiroshisa, 3-3-708, Miyano-cho, Takatsuki-shi Osaka (JP)**
Inventor: **Sasaki, Yutaro, 11-88-504, Okayamate-cho, Hirakato-shi Osaka (JP)**

(74) Representative: **Bentham, Stephen et al, J.A. Kemp & Co. 14 South Square Gray's Inn, London WC1R 5EU (GB)**

(54) **Prostaglandins.**

(57) Prostaglandin D analogues of the formula:

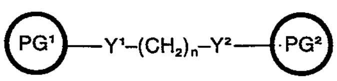

(I)

wherein

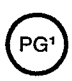 and 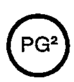 ,

being the same or different, represent groups of the formula:

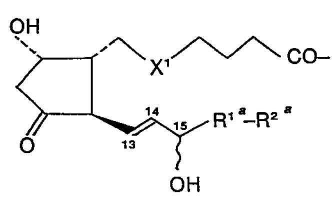

(II)

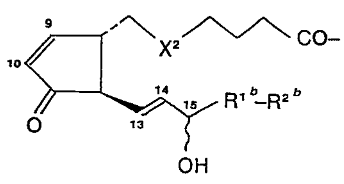

(III)

or

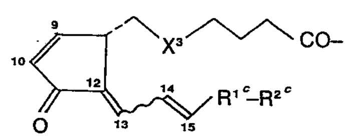

(IV)

wherein $X^1$, $X^2$ and $X^3$, being the same or different, are each ethylene or cis-vinylene, $R^{1a}$, $R^{1b}$ and $R^{1c}$, being the same or different, are each a single bond or alkylene group, $R^{2a}$, $R^{2b}$ and $R^{2c}$, being the same or different are each alkyl, cycloalkyl, phenyl or phenoxy, the double bond between $C_9$–$C_{10}$ is Z, that between $C_{13}$–$C_{14}$ is E, and those between $C_{12}$–$C_{13}$ and $C_{14}$–$C_{15}$, being the same or different, are E, Z or EZ, with the proviso that when $R^{1a}$, $R^{1b}$ or $R^{1c}$ represent a single bond, $R^{2a}$, $R^{2b}$ or $R^{2c}$ respectively do not represent substituted or unsubstituted phenoxy, both $Y^1$ and $Y^2$ are oxygen or imino, or $Y^1$ is imino and $Y^2$ is oxygen, and n is 2 to 8, and cyclodextrin clathrates thereof; possess anti-tumour activity, have an effect of increasing the number of white blood cells and have a synchronisation effect on the cell cycle of tumour cells.

EP 0 172 962 A1

DESCRIPTION

This invention relates to new prostaglandin D analogues, processes for their preparation and pharmaceutical compositions containing them.

Prostaglandins are derivatives of prostanoic acid having the following structure:

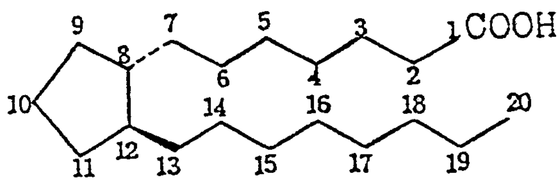

Various types of prostaglandins are known, and their types depend on the structure of the alicyclic ring and the substituents. For example, the alicyclic rings of prostaglandins F (PGF), E (PGE) and D (PGD) have the following structures, respectively:

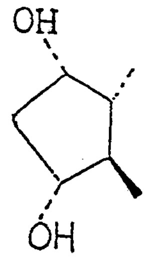

(PGF) ,

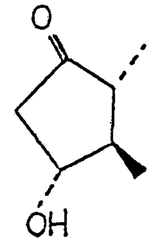

(PGE) and

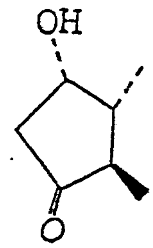

(PGD)

In the above structural formulae or in the other structural formulae in this specification, according to the generally accepted nomenclature, the dotted line indicates that the substituent attached thereto is behind the ring plane, i.e. is of the α-configuration, the bold line indicates that the substituent attached thereto is in front of the ring plane, i.e. is of the ß-configuration, and the wavy line indicates that the substituent attached thereto is of the α-configuration or the ß-configuration or a mixture thereof.

These compounds are sub-classified according to the positions of the double bonds in the side chains attached to the alicyclic ring at the 8-position and the 12-position. The PG-1 compound has a trans-double bond (trans-$\Delta^{13}$) between $C_{13}$-$C_{14}$ and PG-2 compound has a cis-double bond between $C_5$-$C_6$ and a trans-double bond between $C_{13}$-$C_{14}$ (cis-$\Delta^5$, trans-$\Delta^{13}$). For example, prostaglandin $D_1$ ($PGD_1$) and prostaglandin $D_2$ ($PGD_2$) may be expressed by the following structural formulae, respectively:

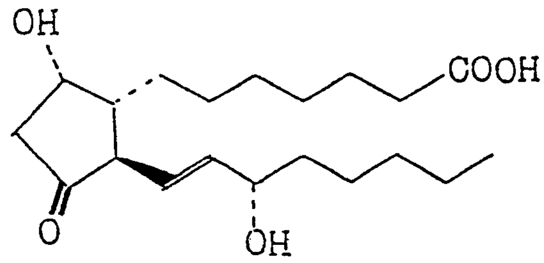

($PGD_1$)

and

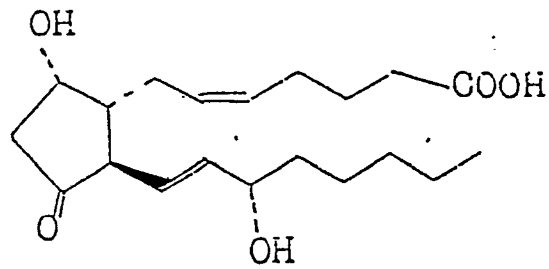

($PGD_2$)

Further, when one or more methylene groups are removed from the aliphatic group attached at the 12-position of the alicyclic ring of a prostaglandin, said compound is known as a nor-prostaglandin according to the general rule of the organic nomenclature, and the number of the removed methylene groups is indicated by adding di-, tri- , etc. before the prefix "nor".

The prostaglandins generally have pharmacological properties. For example, they exert various effects, including the stimulation of contraction of smooth muscles, a hypotensive effect, a diuretic effect, a bronchial dilation effect, the inhibition of lipolysis, the inhibition of platelet aggregation and the inhibition of gastric acid secretion. Therefore, they are useful in treatments of hypertension, thrombosis, asthma and gastric and intestinal ulcers, in the induction of labour and abortion in pregnant mammals, in the prevention of arteriosclerosis and also as diuretics. They are liposoluble substances present in extremely small quantities in the tissues which secrete prostaglandins *in vivo* in animals.

As a result of research and experimentation to discover novel compounds which have the pharmacological effects of the "natural" prostaglandins, or which have one or more of these properties to an enhanced degree, or which have properties which are not found in the "natural" prostaglandins, it has been discovered that certain novel PGD analogues which are obtained by the dimerization, by an optional combination, of $PGD_1$ and $PGD_2$, compounds in which the carboxyl group attached to the 1-position thereof is replaced by a carbamoyl group (PGD amide compounds), compounds in which the hydroxy group attached to the 9-position of the said PGD

compounds or PGD amide compounds is dehydrated to introduce a double bond between $C_9$-$C_{10}$ (9-deoxy-$\Delta^9$-PGD compounds), and further compounds in which the hydroxy group attached to the 15-position of the said 9-deoxy-$\Delta^9$-PGD compounds is dehydrated to introduce double bonds between $C_{12}$-$C_{13}$ and $C_{14}$-$C_{15}$, have strong specific anti-tumor effect.

Heretofore, there have been filed several patent applications relating to PGD analogues (see European Patent Publication Nos. 97023, 98141, 99672 and 99673). However, the dimers of PGD analogues of the present invention are completely novel derivatives which are so far undiscovered and which are unexpected by the conventional techniques.

Furthermore, it has been found that the compounds of the present invention have the effect of increasing the number of white blood cells *in vivo* and have a synchronization effect on the cell cycle of tumour cells.

The present invention provides PGD analogues of the general formula:

$$(PG^1)—Y^1-(CH_2)_n-Y^2—(PG^2) \qquad (I)$$

[wherein $(PG^1)$ and $(PG^2)$, which may be the same or different, each represent a grouping of the general formula,

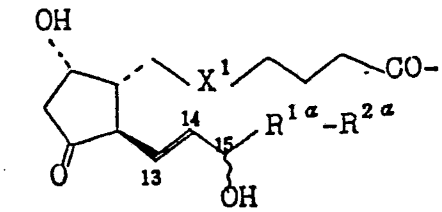

(II),

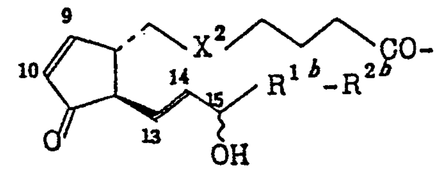

(III)

or .

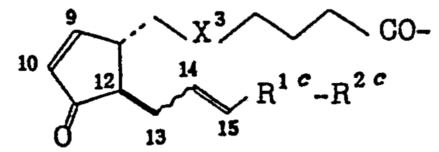

(IV)

(wherein $X^1$, $X^2$ and $X^3$, which may be the same or different, each represents an ethylene group (i.e., $-CH_2CH_2-$) or <u>cis</u>-vinylene group (i.e., $_{H}\!\!>\!C{=}C\!<\!\!_{H}$), $R^{1a}$, $R^{1b}$ and $R^{1c}$, which may be the same or different each represents a single bond or a straight-chain or branched-chain alkylene group of 1 to 5 carbon atoms, $R^{2a}$, $R^{2b}$, and $R^{2c}$, which may be the same or different, each represents a straight-chain or branched-chain alkyl group of 1 to 8 carbon atoms or a cycloalkyl group of 4 to 7 carbon atoms either unsubstituted or substituted by at least one straight-chain or branched-chain alkyl group of 1 to 8 carbon atoms or a phenyl or phenoxy group either unsubstituted or substituted by at least one halogen atom, trifluoromethyl group or straight-chain or branched-chain alkyl group of 1 to 4 carbon atoms, the double bond between $C_9$-$C_{10}$ in the formulae (III)

and (IV) is Z, the double bond between $C_{13}$-$C_{14}$ in the formulae (II) and (III) is E, the double bonds between $C_{12}$-$C_{13}$ and between $C_{14}$-$C_{15}$ in the formula (IV), which may be in the same or different configuration are E, Z or a mixture thereof (i.e., EZ), with the proviso that when $R^{1a}$, $R^{1b}$ or $R^{1c}$ represent a single bond, $R^{2a}$,$R^{2b}$ or $R^{2c}$,respectively,do not represent a substituted or unsubstituted phenoxy group), both $Y^1$ and $Y^2$ represent an oxygen atom, or an imino group (i.e., -NH- group), or $Y^1$ represents an imino group and $Y^2$ represents an oxygen atom and n represents an integer of from 2 to 8],and cyclodextrin clathrates thereof.

In the compounds of the general formula (I), there are several asymmetric carbon atoms. For example, in the general formula (I), wherein (PG¹) or (PG²) represents a group of the formula (II), there exist four asymmetric carbon atoms (at the 8-, 9-, 12- and 15-positions), in the compounds in which (PG¹) or (PG²) is the group represented by the formula (III), the carbon atoms at the 8-, 12- and 15-positions are asymmetric, and in the compounds in which (PG¹) or (PG²) is the group represented by formula (IV), the carbon atom at the 8-position is asymmetric. Further, asymmetric carbon atoms may occur in the substituted groups represented by $R^{1a}$, $R^{1b}$,

$R^{1c}$ and in $R^{2a}$,$R^{2b}$, $R^{2c}$ when the alkyl moiety is branched chain. However, the compounds represented by general formula (I) of the present invention comprise all the isomers and mixtures thereof arising from the presence of asymmetric carbon atoms.

In the general formula (I), (PG$^1$) and (PG$^2$) may be the same or different. Therefore, the combination of the groups of the general formulae (II), (III) and (IV) represented by (PG$^1$) and (PG$^2$) includes eg, (II) and (II), (III) and (III), (IV) and (IV), (II) and (III), (II) and (IV),and (III)and (IV). Preferably, both (PG$^1$) and (PG$^2$) represent the group of the general formula (II) or the group of the general formula (III) or the group of the general formula (IV).

In the general formula (I), there are cases in which both $Y^1$ and $Y^2$ represent an oxygen atom or an imino group, or $Y^1$ represents an imino group and $Y^2$ represents an oxgen atom. Any of these case is considered preferable.

In the general formula (I), the straight-chain alkylene groups of 2 to 8 carbon atoms represented by $(CH_2)_n$ include ethylene, trimethylene, tetramethylene, pentamethylene, hexamethylene, heptamethylene, octamethylene; $(CH_2)_n$ is preferably a straight-chain alkylene group of 2 to 5 carbon atoms.

In the general formulae (II), (III) and (IV), the alkylene groups of 1-5 carbon atoms represented by $R^{1a}$, $R^{1b}$ and $R^{1c}$ include methylene, ethylene, trimethylene, tetramethylene and pentamethylene groups and isomers thereof; $R^{1a}$, $R^{1b}$ and $R^{1c}$ preferably represent a single bond or a methylene or ethylene group.

In the general formulae (II), (III) and (IV), the alkyl groups of 1-8 carbon atoms represented by $R^{2a}$, $R^{2b}$ and $R^{2c}$ include methyl, ethyl, propyl, butyl, pentyl, hexyl, heptyl and octyl groups and isomers thereof; butyl, pentyl or hexyl groups either unsubstituted or substituted by one or two methyl or ethyl groups are preferred.

In the general formulae (II), (III) and (IV), the substituted or unsubstituted cycloalkyl groups represented by $R^{2a}$, $R^{2b}$ and $R^{2c}$ include cyclobutyl, cyclopentyl, cyclohexyl and cycloheptyl groups, and cyclobutyl, cyclopentyl, cyclohexyl and cycloheptyl groups substituted by one or more methyl, ethyl, propyl, butyl, pentyl, hexyl, heptyl or octyl groups or isomers thereof; cyclobutyl, cyclopentyl or cyclohexyl groups either unsubstituted or substituted by one methyl, ethyl, propyl or butyl group are preferred.

In the general formulae (II), (III) and (IV), the substituted or unsubstituted phenyl or phenoxy groups represented by $R^{2a}$, $R^{2b}$ and $R^{2c}$ include phenyl and phenoxy groups, and phenyl and phenoxy groups substituted by one or more atoms or groups selected from the fluorine and chlorine atoms and trifluoromethyl, methyl, ethyl, propyl or butyl

groups; phenyl or phenoxy groups either unsubstituted or substituted by one chlorine atom, trifluoromethyl group, methyl or ethyl group are preferred.

In the general formulae (II), (III) and (IV), preferred groupings $R^{1a}-R^{2a}$, $R^{1b}-R^{2b}$ and $R^{1c}-R^{2c}$ are butyl, pentyl, 1-methylpentyl, 2-methylpentyl, 3-methylpentyl, 1,1-dimethylpentyl, 1-ethylpentyl, 2-ethylpentyl, hexyl, 1-methylhexyl, 2-methylhexyl, 1-ethylhexyl, 2-ethylhexyl, heptyl, 2-methylheptyl, 2-ethylheptyl, cyclobutyl, 1-propylcyclobutyl, 1-butylcyclobutyl, 3-ethylcyclobutyl, 3-propylcyclobutyl, cyclopentyl, cyclopentylmethyl, 2-cyclopentylethyl, 3-ethylcyclopentyl, 3-propylcyclopentyl, 3-butylcyclopentyl, cyclohexyl, cyclohexylmethyl, 2-cyclohexylethyl, 4-methylcyclohexyl, 4-ethylcyclohexyl, 4-propylcyclohexyl, 4-butylcyclohexyl, benzyl, 2-phenylethyl, 4-methylbenzyl, 4-ethylbenzyl, phenoxymethyl, 2-phenoxyethyl, 3-chlorophenoxymethyl, 4-chlorophenoxymethyl, 3-trifluoromethylphenoxymethyl, 4-trifluoromethylphenoxymethyl, 4-methylphenoxymethyl and 4-ethylphenoxymethyl; n-pentyl, 1-methylpentyl, 1,1-dimethylpentyl, 2-methylhexyl, 1-butylcyclobutyl, cyclopentyl, 3-propylcyclopentyl, 4-butylcyclohexyl, benzyl, phenoxymethyl, 3-chlorophenoxymethyl or (3-trifluoromethyl)phenoxymethyl are especially preferred.

Further, in the general formulae (II), (III) and (IV) $X^1$, $X^2$ and $X^3$ are preferably a <u>cis</u>-vinylene group, and in the

general formulae (II) and (III), the preferred configuration of the hydroxyl group attached to the carbon atom at the 15-position is the α-configuration.

In the general formulae (II), (III) and (IV), $X^1$, $X^2$ and $X^3$, $R^{1a}$, $R^{1b}$ and $R^{1c}$, and $R^{2a}$, $R^{2b}$ and $R^{2c}$ may be the same or different. This means that such groups may be the same or different to each other, when (PG$^1$) and (PG$^2$) represent any of the groups of the general formulae (II), (III) and (IV). For example, if both (PG$^1$) and (PG$^2$) are represented by the general formula (II), they have $X^1$, $R^{1a}$ and $R^{2a}$ in common. In this case, pairs of $X^1$, $R^{1a}$ and $R^{2a}$ may be the same or different. When (PG$^1$) represents a group of the general formula (II) and (PG$^2$) represents a group of the general formula (III), there exist $X^1$ and $X^2$, $R^{1a}$ and $R^{1b}$, and $R^{2a}$ and $R^{2b}$: these may be the same or different. Preferably, $X^1$, $X^2$ and $X^3$, $R^{1a}$, $R^{1b}$ and $R^{1c}$, and $R^{2a}$, $R^{2b}$ and $R^{2c}$, respectively, represent the same groups.

According to the present invention, the PGD analogues of the general formula (I), wherein the various symbols are as hereinbefore defined can be prepared by reacting the PGD analogues of the general formula:

$$(PG^1)-OH \qquad (V)$$

(wherein (PG$^1$) is as hereinbefore defined) with the compounds of the general formulae:

$$HO-(CH_2)_n-OH \qquad (VI)$$

$$H_2N-(CH_2)_n-OH \qquad (VII)$$

or

$$H_2N-(CH_2)_n-NH_2 \qquad (VIII)$$

(wherein n is as hereinbefore defined) to obtain a compound of the general formula:

$$(PG^1)-Y^1-(CH_2)_n-Y^2-H \qquad (IX)$$

(wherein the various symbols are as hereinbefore defined) and then reacting the resulting compound of general formula IX with the PGD analogues of the general formula:

$$(PG^2)-OH \qquad (X)$$

(wherein the various symbols are as hereinbefore defined).

The reaction of the compounds of the general formula (V) with the compounds of the general formula (VI), (VII) or (VIII) can be conducted by reacting the compounds of the general formula (V) with isobutyl chloroformate or

trimethylacetyl chloride in an inert organic solvent, for example, methylene chloride, tetrahydrofuran or N,N-dimethylformamide, in the presence of a base such as triethylamine or pyridine, at a temperature of from 0°C to room temperature, to form an anhydrous acid mixture of the compounds of the general formula (V), and by reacting it with the compounds of the general formulae (VI), (VII) or (VIII) at a temperature of from 0°C to room temperature. (This sequence of the reaction steps is hereinafter referred to as "Reaction A").

In this reaction, it is preferable that the compounds of the general formulae (VI), (VII) or (VIII) exceed in quantity the compounds of the general formula (V), preferably at the rate of 8 to 12 mols of the former to 1 mol of the latter.

The compound of general formula (IX) is then reacted with the compounds of the general formula (X) under the conditions for Reaction A described hereinbefore.In this case, it is advisable to use the compounds of the general formula (X) and compounds of the general formula (IX) at the rate of 1 mol of the former to 0.5 to 1.0 mols of the latter.

In the compounds of the general formula (VII), the amino group is more reactive than the hydroxy group. Therefore, to prepare compounds of the general formula (I)

wherein $Y^1$ represents an imino group and $Y^2$ represents an oxygen atom, it is essential to react PGD analogues which are needed to be bonded with an imino group, first with compounds of the general formula (VII).

The compounds of the general formula (IX) wherein both $Y^1$ and $Y^2$ represent an imino group can be isolated and purified in a form of a salt with a suitable acid (e.g., hydrochloric acid).

The compounds of the general formulae (VI), (VII) and (VIII) are known products in themselves or can be prepared according to the known processes.

The compounds of the general formula (I), wherein $(PG^1)$ and $(PG^2)$, which may be the same or different, each represents a group of the formula (II) or (III), $Y^1$ and $Y^2$ both represent an oxygen atom or $Y^1$ represents an imino group and $Y^2$ represents an oxygen atom, and the other symbols are as hereinbefore defined, that is, the compounds of the general formula:

$$(PG^3) - Y^3 - (CH_2)_n - Y^4 - (PG^4) \qquad (IA)$$

{wherein $(PG^3)$ and $(PG^4)$, which may be the same or different, each represents a group of the general formula (II) or (III)

(wherein the various symbols are as hereinbefore defined), $Y^3$ and $Y^4$ both represent an oxygen atom, or $Y^3$ represents an imino group and $Y^4$ represents an oxygen atom, and n is as hereinbefore defined] can be prepared by hydrolyzing, under acidic conditions, the compounds of the general formula

$$(PG^3) — Y^3 - (CH_2)_n - Y^4 — (PG^4) \quad (XI)$$

[wherein $(PG^3)$ and $(PG^4)$, which may be the same or different, each represents a group of the general formula:

(XII)

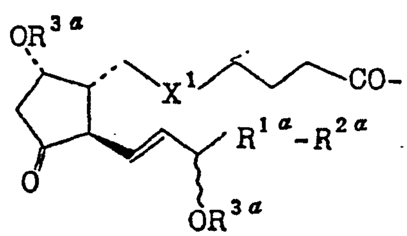

or

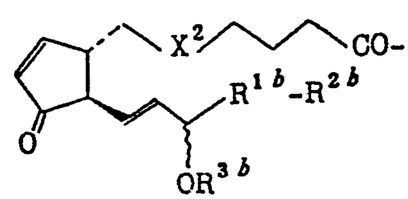

(XIII)

(wherein $R^{3a}$ and $R^{3b}$, which may be the same or different, each represents a tetrahydropyran-2-yl group or tetrahydrofuran-2-yl group either unsubstituted or substituted by at least one alkyl group, or a 1-ethoxyethyl group, preferably a tetrahydropyran-2-yl group, and the other symbols are as hereinbefore defined), and the other symbols are as hereinbefore defined]. This reaction is generally conducted:

(1) In an aqueous solution of an organic acid such as acetic

acid, propionic acid, oxalic acid or p-toluenesulfonic acid or an aqueous solution of an inorganic acid such as hydrochloric acid, sulfuric acid or phosphoric acid, suitably in the presence of a water-miscible organic solvent, for example, a lower alkanol such as methanol or ethanol (preferably methanol) or an ether such as 1,2-dimethoxyethane, dioxane or tetrahydrofuran (preferably tetrahydrofuran) at a temperature of from room temperature to 80°C, or

(2) in an anhydrous lower alkanol such as methanol or ethanol, in the presence of an organic acid such as p-toluenesulfonic acid or trifluoroacetic acid at a temperature of 10 to 45°C.

The hydrolysis is preferably conducted using a mixture of dilute hydrochloric acid and tetrahydrofuran, a mixture of dilute hydrochloric acid and methanol, a mixture of acetic acid, water and tetrahydrofuran, a mixture of phosphoric acid, water and tetrahydrofuran, or a mixture of p-toluenesulfonic acid and anhydrous methanol.

The compounds of the general formula (XI) can be prepared, by the sequence of reactions hereinbefore described for the preparation of compounds of general formula (I) but replacing the compounds of formula (V) and (X) by compounds of the general formulae:

$(PG^3)$— OH (XIV)

and

$(PG^4)$— OH (XV)

(wherein the various symbols are as hereinbefore defined) and using the compounds of the general formulae (VI) or (VII).

The present method provides, in principle, processes for the preparation of dimers of PGD analogues by dimerizing PGD analogues protected by a protective group and then eliminating the protective group. According to a feature of the invention the process can be conducted also by using a PGD analogue not protected by a protecting group, i.e. $(PG^1)$—OH or $(PG^2)$—OH as starting material instead of either $(PG^3)$—OH or $(PG^4)$—OH.

Furthermore, according to the present invention, the PGD analogues of the general formula (I) wherein $(PG^1)$ and $(PG^2)$ are identical, i.e. the PGD analogues of the general formula:

$$\text{(PG}^5\text{)} - Y^1 - (CH_2)_n - Y^2 - \text{(PG}^5\text{)} \qquad (IB)$$

[wherein both groups (PG$^5$), which are the same, represent a group of the general formula:

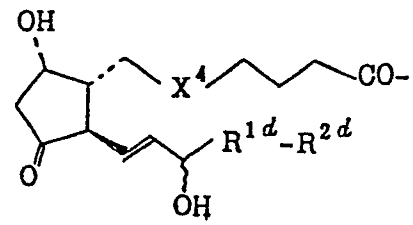

(IIa),

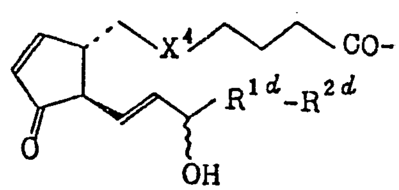

(IIIa)

or

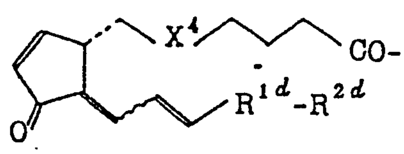

(IVa);

(wherein $X^4$ represents an ethylene group or a cis-vinylene group, $R^{1d}$ represents a single bond or a straight-chain or branched-chain alkylene group of 1 to 5 carbon atoms, $R^{2d}$ represents a straight-chain or branched-chain alkyl group of 1 to 8 carbon atoms, a cycloalkyl group of 4 to 7 carbon atoms either unsubstituted or substituted by at least one straight-chain or branched-chain alkyl group of 1 to 8 carbon atoms, or a phenyl or phenoxy group either unsubstituted or substituted by at least one halogen atom, trifluoromethyl group or straight-chain or branched-chain alkyl group of 1 to 4 carbon atoms, the double bond between $C_9$-$C_{10}$ in the general formulae (IIIa) and (IVa) is Z, the double bond between $C_{13}$-$C_{14}$ in the general formulae (IIa) and (IIIa) is E, the double bonds between $C_{12}$-$C_{13}$ and $C_{14}$-$C_{15}$ in the general formula (IVa), which may be in the same or different configurations are E, Z or EZ, with the proviso that when $R^{1d}$ represents a single bond, $R^{2d}$ does not represent a substituted or unsubstituted phenoxy group) and the other symbols are as hereinbefore defined] can be prepared by reacting a PGD analogue of the general formula:

$$(PG^5)\text{— OH} \qquad \text{(XVI)}$$

(wherein $PG^{5}$ is as hereinbefore defined) with a compound of the general formula (VI), (VII) or (VIII), in one process, according to the steps of Reaction A hereinbefore described.

In this reaction, it is preferable to use 1/2 mol of the compound of the general formula (VI), (VII) or (VIII) to 1 mol of the compound of the general formula (XVI).

Furthermore, the compounds of the general formula (IB) in which $PG^{5}$ represents a group of the general formulae (IIa) or (IIIa), that is the compound of the general formula:

$$PG^{6} - Y^{1} - (CH_{2})_{n} - Y^{2} - PG^{6} \qquad (IC)$$

[wherein both groups $PG^{6}$, which are the same, represent a group of the general formula:

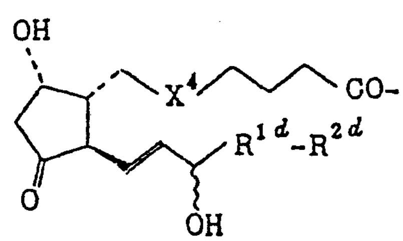

(IIa)

or

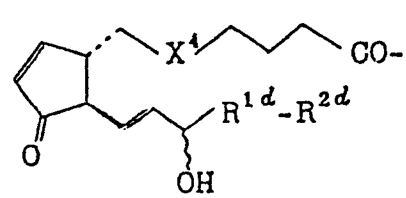

(IIIa)

(wherein the various symbols are as hereinbefore defined) and the other symbols are as hereinbefore defined] can be prepared by the hydrolysis, under acidic conditions, of a compound of the general formula:

$$(PG^{6'}) - Y^1 - (CH_2)_n - Y^2 - (PG^{6'}) \qquad \text{(XVII)}$$

[wherein both groups $(PG^{6'})$, which are the same, represent a group of the formula:

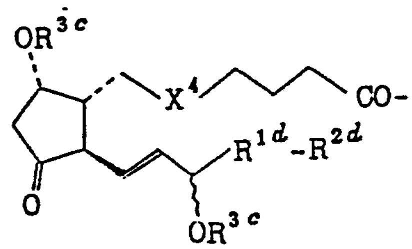

(XVIII)

or

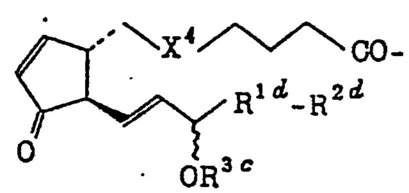

(XIX)

(wherein the groups $R^{3c}$ are identical and each represents a tetrahydropyran-2-yl group or a tetrahydrofuran-2-yl group either unsubstituted or substituted by at least one alkyl group or a 1-ethyoxyethyl group; preferably a tetrahydropyran-2-yl group, and the other symbols are as hereinbefore defined) and the other symbols are as hereinbefore defined].

This hydrolysis is preferably conducted by the method hereinbefore described for the conversion of compounds of general formula (XI) to those of general formula (IA).

The compounds of the general formula (XVII) can be prepared, by the process hereinbefore described for the preparation of compounds of general formula (IB) but replacing the compound of general formula (XVI) by a compound of the general formula:

$$\textcircled{PG^{6}} — OH \qquad (XX)$$

(wherein $\textcircled{PG^{6}}$ is as hereinbefore defined).

According to a further feature of the present invention, the PGD analogues of the general formula (I) in which $\textcircled{PG^{1}}$ and $\textcircled{PG^{2}}$ both represent a group of the general formula (IV) (wherein pairs of $X^3$, $R^{1c}$ and $R^{2c}$ in $\textcircled{PG^{1}}$ and $\textcircled{PG^{2}}$ may be the same or different), that is, the PGD analogues

represented by the general formula:

$$(PG^7) - Y^1 - (CH_2)_n - Y^2 - (PG^8) \qquad \text{(ID)}$$

[wherein $(PG^7)$ and $(PG^8)$ each represents a group represented by the general formula:

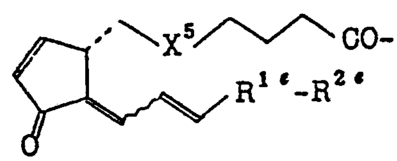

(XXI)

(wherein $X^5$ represents an ethylene group or a cis-vinylene group, $R^{1e}$ represents a single bond or a straight-chain or branched-chain alkylene group of 1 to 5 carbon atoms, $R^{2e}$ represents a straight-chain or branched-chain alkyl group of 1 to 8 carbon atoms, a cycloalkyl group of 4 to 7 carbon atoms either unsubstituted or substituted by at least one straight-chain or branched-chain alkyl group of 1 to 8 carbon atoms, or a phenyl or phenoxy group either unsubstituted or substituted by at least one halogen atom, trifluoromethyl group or straight-chain or branched-chain alkyl group of 1 to

4 carbon atoms, the double bond between $C_9$-$C_{10}$ is Z, and the double bonds between $C_{12}$-$C_{13}$ and $C_{14}$-$C_{15}$, which may be in the same or different configuration, are E, Z or EZ, with the proviso that when $R^{1e}$ represents a single bond, $R^{2e}$ does not represent a substituted or unsubstituted phenoxy group and pairs of $X^5$, $R^{1e}$ and $R^{2e}$ in $(PG^7)$ and $(PG^8)$ may be the same or different) and the other symbols are as hereinbefore defined] can be prepared by the reaction of a compound of the general formula:

$$(PG^3) - Y^1 - (CH_2)_n - Y^2 - (PG^4) \quad \text{(IF)}$$

or

$$(PG^3) - Y^3 - (CH_2)_n - Y^4 - (PG^4) \quad \text{(XI)}$$

(wherein the various symbols are as hereinbefore defined) with an aqueous acid at the reflux temperature. This reaction is preferably conducted in an inert organic solvent, for example, methylene chloride, chloroform, carbon tetrachloride, diethyl ether, N,N-dimethylformamide, tetrahydrofuran or a mixture of two or more such solvents, using as the aqueous acid, for example, an aqueous solution of an inorganic acid, e.g., hydrochloric acid, hydrobromic acid, sulfuric acid or phosphoric acid or an organic acid, e.g., acetic acid, propionic acid or oxalic acid, at the reflux temperature of the solvent. The reaction is preferably carried out in tetrahydrofuran using 1 N

hydrochloric acid at the reflux temperature of the reaction mixture. According to a further feature of the invention the compounds of the general formula (ID) can also be prepared by the reaction of a compound of the general formula (IF), with an alkylsulphonyl chloride or arylsulphonyl chloride, generally in a suitable organic solvent, in the presence of a base such as triethylamine, at a low temperature, preferably at a temperature of from -30°C to 0°C. The alkylsulphonyl chloride preferably contains from 1 to 4 carbon atoms in the alkyl group (e.g. methanesulphonyl chloride). Tosyl chloride is the preferred arylsulphonyl chloride.

In the processes of the present invention, the compounds of the general formulae (V), (X), (XIV), (XV), (XVI) and (XX) used as starting materials can be produced according to the processes described in the specifications of British Patent No. 1403838 and United States Patent No. 4016184. These compounds can also be produced according to the sequence of reaction steps illustrated in Scheme A. In the scheme, X represents $X^1$, $X^2$, $X^3$ or $X^4$, $R^1$ represents $R^{1a}$, $R^{1b}$, $R^{1c}$, or $R^{1d}$, $R^2$ represents $R^{2a}$, $R^{2b}$, $R^{2c}$ or $R^{2d}$, $R^3$ represents $R^{3a}$, $R^{3b}$ or $R^{3c}$, $R^4$ represents an acyl group (For example, an acetyl group or benzoyl group), $R^5$ represents an alkylsulphonyl group or arylsulphonyl group (for example, a mesyl group or tosyl group) and the other symbols are as hereinbefore defined.

Scheme A

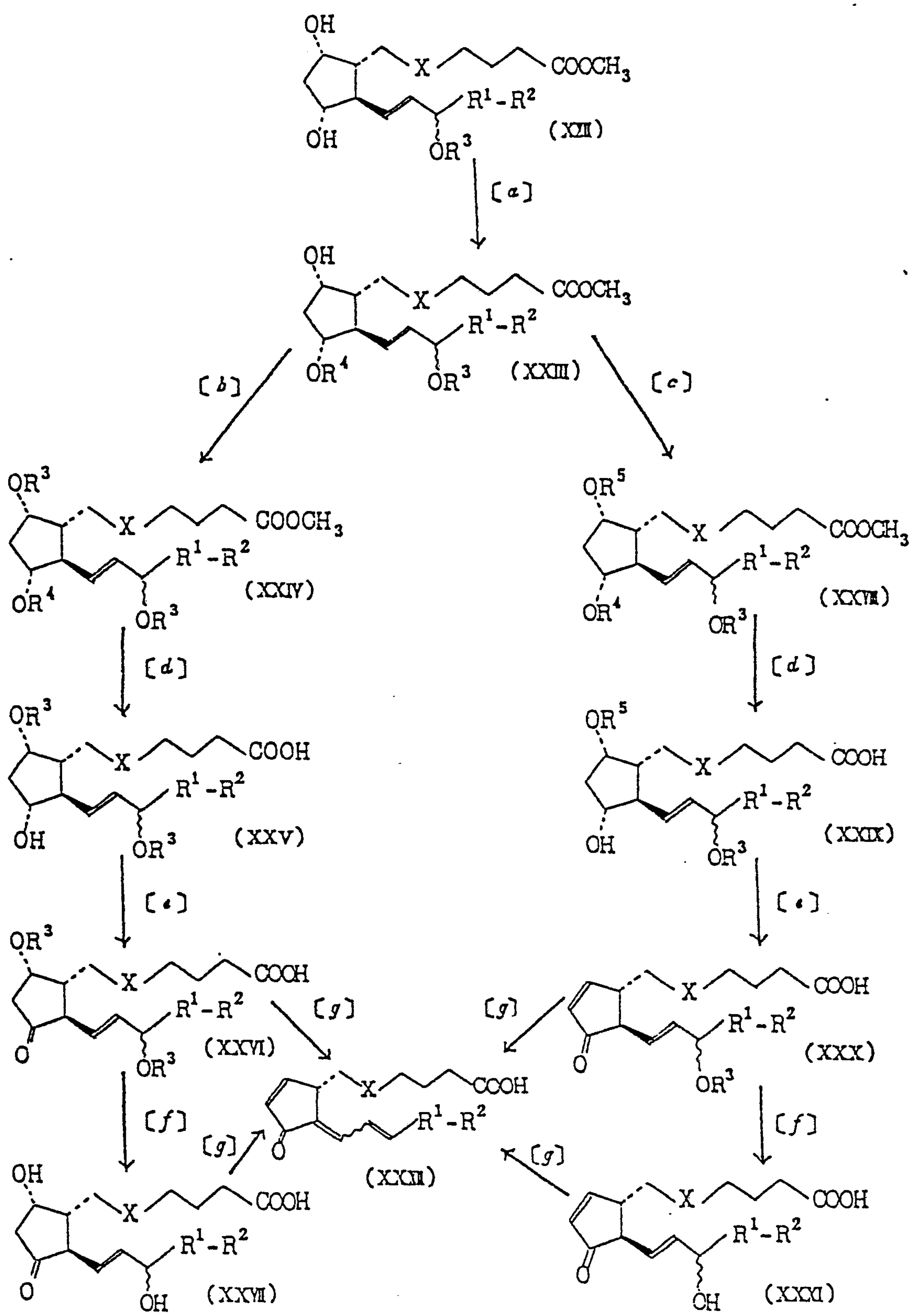


(XXII) (XXIII) (XXIV) (XXVIII) (XXV) (XXIX) (XXVI) (XXX) (XXXII) (XXVII) (XXXI)

All the steps in the Scheme are conducted by known methods, for example, as described below. The step (a) may be conducted by selectively acylating the hydroxyl group attached to the 11-position of the compounds of the general formula (XXII). Such acylation may be conducted using an appropriate acyl chloride or acid anhydride, for example, benzoyl chloride, in an inert organic solvent, for example, methylene chloride, or in the absence of a solvent in the presence of a tertiary amine such as pyridine or triethylamine at a temperature not higher than room temperature, preferably at -30°C to -40°C. The step (b) may be conducted using, e.g., 2,3-dihydropyran, 2,3-dihydrofuran or ethyl vinyl ether, in an inert organic solvent, for example, methylene chloride, chloroform or diethyl ether, in the presence of a condensing agent, for example, p-toluenesulfonic acid, sulfuric acid or trifluoroacetic acid at a temperature of from room temperature to -30°C. Preferably, it is conducted using 2,3-dihydropyran in methylene chloride in the presence of pyridine p-toluenesulfonate or p-toluenesulfonic acid at room temperature.

The step (c) may be conducted by reacting with an alkylsulfonyl chloride such as mesyl chloride or arylsulfonyl chloride such as tosyl chloride (i) in an inert organic solvent such as methylene chloride, in the presence of a

tertiary amine such as pyridine or triethylamine or (ii) in pyridine, at a temperature of from -30°C to 50°C.

The step (d) may be conducted by deacylation with a hydroxide of an alkali metal such as lithium, sodium or potassium in an aqueous lower alkanol such as aqueous methanol or aqueous ethanol at a temperature of from 0 to 60°C.

The step (e) is a reaction to convert the hydroxy group into an oxo group and, in addition to eliminate the $OR^5$ group to form a double bond between $C_9$-$C_{10}$. Such an oxidation reaction is well known, and is described in detail in, for example,

(a) "Synthetic Organic Chemistry III, Organic Synthesis 1", pp. 176-206 (compiled by Tetsuji Kameya and published by Nankodo (Japan) on Aug. 1, 1976), or,

(b) "Compendium of Organic Synthetic Methods", vol. 1, vol. 2, and vol.3, section 48 or 168 (published by John Wiley & Sons, Inc. (USA) in 1971, 1974, and 1977, respectively).

The oxidation is preferably carried out under mild neutral conditions using, for example, dimethyl-sulphide-N-chlorosuccinimide complex, thioanisole-N-chlorosuccinimide complex, dimethylsulphide-chlorine complex, thioanisole-chlorine complex (see J. Amer. Chem. Soc., 94, 7586 (1972) with respect to these complexes), dicyclohexylcarbodiimide-dimethylsulphoxide complex (see J. Amer. Chem. Soc., 87, 5661 (1965)), pyridinium chlorochromate ($C_5H_5NHCrO_3Cl$) (see Tetrahedron Letters, 2647 (1975)), sulphuric anhydride-pyridine complex (see J. Amer. Chem. Soc., 89, 5505 (1967)), chromyl chloride (see. J. Amer. Chem. Soc., 97, 5929 (1975)), chromium trioxide-pyridine complex (for example, Collins' reagent), Jones' reagent or chromic acid solution (prepared from chromium trioxide, manganese sulfate, sulfuric acid and water), or oxalyl chloride and dimethylsulfoxide (i.e. Swern oxidation); suitably the Collins oxidation, Jones oxidation or Swern oxidation may be employed. The Collins oxidation may be conducted in a halogenated hydrocarbon such as chloroform, methylene chloride or carbon tetrachloride at a temperature of from room temperature to 0°C, preferably at 0°C. The Jones oxidation is generally conducted at a temperature of not higher than room temperature. The Swern oxidation may be conducted by reaction in a halogenated hydrocarbon such as chloroform or methylene chloride at a temperature of from -50°C to -60°C, and then treatment with triethylamine.

The step (f) may be carried out by the method hereinbefore described for the conversion of compounds of general formula (XI) to those of general formula (IA).

The step (g) may be carried out by the method hereinbefore described for the conversion of compounds of general formula (IF) or (XI) to those of general formula (ID).

The compounds of the general formula (XXII) are known compounds and disclosed as compounds of the general formula (XXIII) in European Patent Publication No. 98141.

The cyclodextrin clathrates of the prostaglandin D analogues of general formula (I) can be prepared using α-, β- or γ-cyclodextrin or mixture thereof, using the procedure described in British Patent Nos. 1351238 and 1419221. Conversion into the cyclodextrin clathrate serves to increase the stability of the prostaglandin D analogue of the general formula (I).

The prostaglandin D analogues of the general formula (I) and their cyclodextrin clathrates have a specific strong anti-tumour effect, and their toxicity is extremely low. They may therefore be employed as very effective anti-tumour agents in the prevention or therapy of leukemia and solid cancer, and in treatment to produce remission thereof.

In addition, prostaglandin D analogues of the general formula (I) have the effect of increasing the number of white blood cells in vivo. Therefore compounds of the present invention are effective as a preventive and therapeutic agent for the infection by such microorganisms as bacteria and viruses. Compounds of the present invention may also be used to treat leukocyto-penia induced by administration of conventional anti-tumour agents, because such anti-tumour agents generally cause

a decrease in the number of white blood cells as a side effect.

Furthermore, prostaglandin D analogues of the general formula (I) have a synchronization effect on the cell cycle of tumour cells. Cells differentiate and proliferate by repeating such cycle as $G_1$ stage (preparative stage for the synthesis of DNA) ⟶ S stage (stage for the synthesis of DNA) ⟶ $G_2$ stage (stage for the synthesis of protein) ⟶ M stage (stage for cell division) ⟶ $G_1$ stage. The compounds of the present invention have the effect of synchronizing tumour cells which exist in four different stages *in vivo*, to the $G_1$ stage. Therefore, when administering a conventional anti-tumour agent which can only attack tumour cells at a particular stage, the compounds of the present invention increase the effect of the said anti-tumour agent.

In an *in vitro* test on the inhibition of proliferation of cells originated from human mouth tumour (KB-cells) the compounds of the present invention showed an excellent anti-tumour effect. The experimental methods and the results are described below.

Test on Inhibition of Proliferation Using Cells Originated from Human Mouth Tumour (KB-cells)

(Experimental Method)

The cells originated from human mouth tumour (KB-cells) were added to Eagle's MEM culture solution containing 10% bovine fetal serum, the number of cells in the culture solution was adjusted to $1 \times 10^5$ cells/ml, a solution in ethanol of compounds of the present invention was added to give a concentration of 5 µg/ml or below and the mixture was subjected

to stationary culture at 37°C for 4 days. As a control, a culture solution containing 0.1% of ethanol was similarly cultured. After the culture, the cells were stained by the Trypan Blue staining method, and the surviving cell number was measured to determine the $IC_{50}$ value from the degree of inhibition relative to the control. The results are given below.

Inhibition of Proliferation Using Cells Originated from Human Mouth Tumour (KB-cells)

| Preparing Example No. of Test Compound | Inhibition of Proliferation ($IC_{50}$, μg/ml) |
|---|---|
| Example 1 | 0.87 |
| Example 1(f) | 1.17 |
| Example 1(g) | 1.8 |
| Example 1(h) | <1 |
| Example 3(a) | 1.9 |
| Example 4 | 0.47 |

In an in vivo test on increase of white blood cells using blood collected from male mice, the compounds of the present invention showed an excellent effect. The experimental methods and results are described below.

Test on increase of white blood cell

(Experimental Method)

A solution of a compound of the present invention in ethanol was diluted with 1% Tween 80 (registered Trade Mark)

and the solution was intraperitoneally administered to ICR male mice of 6-7 weeks old once a day for 14 days. On the day following the final administration, the blood was collected by cardiac puncture with Anglot® (containing heparin and EDTA) as an anticoagulant. The blood was diluted with Seruento (registered Trade Mark), and the numbers of white blood cells and red blood cells were counted by a Micro Cell Counter (model CC-120). The results are given below.

Effect on Hematological Test

| Preparing Example No. of Test Compounds | Dose (mg/kg, i.p.) | WBC ($x10^2/mm^3$) | RBC ($x10^4/mm^3$) |
|---|---|---|---|
| control | | 113 ± 5.6 | 941 ± 10.3 |
| Example 1(e) | 3 | 158 ± 17.0 ($p < 0.05$) | 793 ± 22.5 ($p < 0.001$) |

WBC: white blood cells

RBC: Red blood cells

As shown in the Table, red blood cells decrease by a factor of about 0.8; white blood cells increase about 1.4 times at the dose of 3 mg/kg as compared with control. Therefore, the compounds of the present invention have the effect of increasing the number of white blood cells.

On the other hand, it was confirmed that the acute toxicity of the compounds of the present invention was very weak. For example, the acute toxicity of the compound prepared in Example 1(e) was more than 100 mg/kg intravenously. Therefore, prostaglandin D analogues of the present invention may be considered to be sufficiently safe and suitable for medical use.

Of the compounds represented by general formula (I) which fall within the present invention, examples of preferred compounds include dimers of PGD analogues wherein $Y^1$, $Y^2$, n and $R^1$-$R^2$ are as shown in the Table below in the following general formulae, and the corresponding compounds wherein a cis-vinylene group between $C_5$-$C_6$ thereof is replaced by an ethylene group.

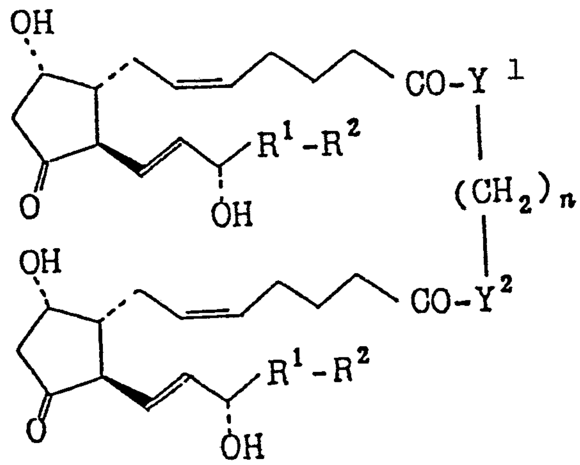

(I-1)、

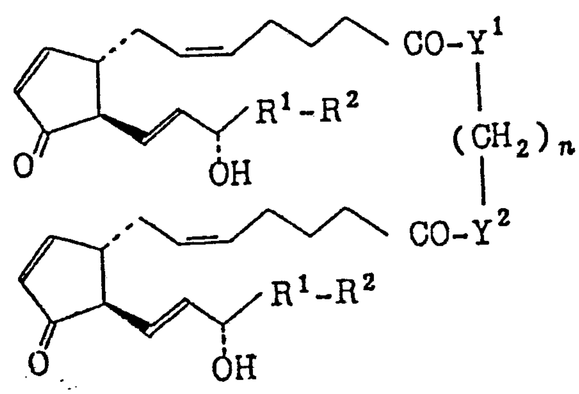

(I-2)

or

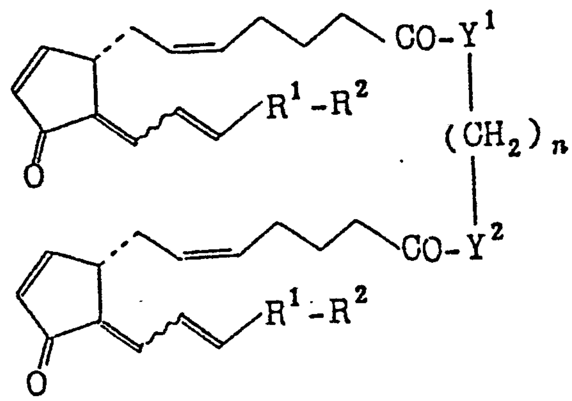

(I-3)

Table

| No. | $Y^1$ | $Y^2$ | n | $R^1$-$R^2$ |
|---|---|---|---|---|
| 1 | O | O | 2 | n-pentyl |
| 2 | " | " | 3 | " |
| 3 | " | " | 4 | " |
| 4 | NH | O | 2 | " |
| 5 | " | " | 3 | " |
| 6 | " | " | 4 | " |
| 7 | NH | NH | 2 | " |
| 8 | " | " | 3 | " |
| 9 | " | " | 4 | " |
| 10 | O | O | 2 | 1-methylpentyl |
| 11 | " | " | 3 | " |
| 12 | " | " | 4 | " |
| 13 | NH | O | 2 | " |
| 14 | " | " | 3 | " |

| No. | $Y^1$ | $Y^2$ | n | $R^1$-$R^2$ |
|---|---|---|---|---|
| 15 | NH | O | 4 | 1-methylpentyl |
| 16 | NH | NH | 2 | " |
| 17 | " | " | 3 | " |
| 18 | " | " | 4 | " |
| 19 | O | O | 2 | 1,1-dimethylpentyl |
| 20 | " | " | 3 | " |
| 21 | " | " | 4 | " |
| 22 | NH | O | 2 | " |
| 23 | " | " | 3 | " |
| 24 | " | " | 4 | " |
| 25 | NH | NH | 2 | " |
| 26 | " | " | 3 | " |
| 27 | " | " | 4 | " |
| 28 | O | O | 2 | 2-methylhexyl |
| 29 | " | " | 3 | " |
| 30 | " | " | 4 | " |
| 31 | NH | O | 2 | " |
| 32 | " | " | 3 | " |

| No. | $Y^1$ | $Y^2$ | $n$ | $R^1$-$R^2$ |
|---|---|---|---|---|
| 33 | NH | O | 4 | 2-methylhexyl |
| 34 | NH | NH | 2 | " |
| 35 | " | " | 3 | " |
| 36 | " | " | 4 | " |
| 37 | O | O | 2 | 1-butylcyclobutyl |
| 38 | " | " | 3 | " |
| 39 | " | " | 4 | " |
| 40 | NH | O | 2 | " |
| 41 | " | " | 3 | " |
| 42 | " | " | 4 | " |
| 43 | NH | NH | 2 | " |
| 44 | " | " | 3 | " |
| 45 | " | " | 4 | " |
| 46 | O | O | 2 | cyclopentyl |
| 47 | " | " | 3 | " |
| 48 | " | " | 4 | " |
| 49 | NH | O | 2 | " |
| 50 | " | " | 3 | " |

| No. | $Y^1$ | $Y^2$ | n | $R^1$-$R^2$ |
|---|---|---|---|---|
| 51 | NH | O | 4 | cyclopentyl |
| 52 | NH | NH | 2 | 〃 |
| 53 | 〃 | 〃 | 3 | 〃 |
| 54 | 〃 | 〃 | 4 | 〃 |
| 55 | O | O | 2 | 3-propylcyclopentyl |
| 56 | 〃 | 〃 | 3 | 〃 |
| 57 | 〃 | 〃 | 4 | 〃 |
| 58 | NH | O | 2 | 〃 |
| 59 | 〃 | 〃 | 3 | 〃 |
| 60 | 〃 | 〃 | 4 | 〃 |
| 61 | NH | NH | 2 | 〃 |
| 62 | 〃 | 〃 | 3 | 〃 |
| 63 | 〃 | 〃 | 4 | 〃 |
| 64 | O | O | 2 | 4-butylcyclohexyl |
| 65 | 〃 | 〃 | 3 | 〃 |
| 66 | 〃 | 〃 | 4 | 〃 |
| 67 | NH | O | 2 | 〃 |
| 68 | 〃 | 〃 | 3 | 〃 |

| No. | $Y^1$ | $Y^2$ | $n$ | $R^1$-$R^2$ |
|---|---|---|---|---|
| 69 | NH | O | 4 | 4-butylcyclohexyl |
| 70 | NH | NH | 2 | 〃 |
| 71 | 〃 | 〃 | 3 | 〃 |
| 72 | 〃 | 〃 | 4 | 〃 |
| 73 | O | O | 2 | benzyl |
| 74 | 〃 | 〃 | 3 | 〃 |
| 75 | 〃 | 〃 | 4 | 〃 |
| 76 | NH | O | 2 | 〃 |
| 77 | 〃 | 〃 | 3 | 〃 |
| 78 | 〃 | 〃 | 4 | 〃 |
| 79 | NH | NH | 2 | 〃 |
| 80 | 〃 | 〃 | 3 | 〃 |
| 81 | 〃 | 〃 | 4 | 〃 |
| 82 | O | O | 2 | phenoxymethyl |
| 83 | 〃 | 〃 | 3 | 〃 |
| 84 | 〃 | 〃 | 4 | 〃 |
| 85 | NH | O | 2 | 〃 |
| 86 | 〃 | 〃 | 3 | 〃 |

| No. | $Y^1$ | $Y^2$ | n | $R^1$-$R^2$ |
|---|---|---|---|---|
| 87 | NH | O | 4 | phenoxymethyl |
| 88 | NH | NH | 2 | " |
| 89 | " | " | 3 | " |
| 90 | " | " | 4 | " |
| 91 | O | O | 2 | 3- chlorophenoxymethyl |
| 92 | " | " | 3 | " |
| 93 | " | " | 4 | " |
| 94 | NH | O | 2 | " |
| 95 | " | " | 3 | " |
| 96 | " | " | 4 | " |
| 97 | NH | NH | 2 | " |
| 98 | " | " | 3 | " |
| 99 | " | " | 4 | " |
| 100 | O | O | 2 | (3-trifluoromethyl)-phenoxymethyl |
| 101 | " | " | 3 | " |
| 102 | " | " | 4 | " |
| 103 | NH | O | 2 | " |
| 104 | " | " | 3 | " |

| No. | $Y^1$ | $Y^2$ | n | $R^1$-$R^2$ |
|---|---|---|---|---|
| 105 | NH | O | 4 | (3-trifluoromethyl)-phenoxymethyl |
| 106 | NH | NH | 2 | 〃 |
| 107 | 〃 | 〃 | 3 | 〃 |
| 108 | 〃 | 〃 | 4 | 〃 |

The following Reference Examples and Examples illustrate the preparation of compounds of the present invention. In the Reference Examples and Examples, "TLC", "NMR" and "IR" represent "Thin layer chromatography", "Nuclear magnetic resonance spectrum" and "Infrared absorption spectrum" respectively. Further, "Ms" and "THP" in the structural formulae represent mesyl group (methanesulfonyl group) and tetrahydropyran-2-yl group, respectively, and "THF" and "AcOH" as solvents represent tetrahydrofuran and acetic acid, respectively.

The solvents in parentheses in "TLC" show the developing solvents used. Unless otherwise specified, "IR" was measured by the liquid film method and "NMR" was measured in a deuterochloroform ($CDCl_3$) solution.

Reference Example 1

Synthesis of:

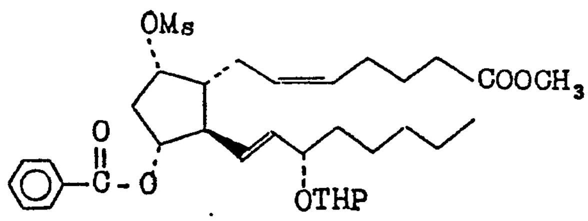

To a solution of 2.8 g of (5Z,13E)-(9α,11α,15S)-9-hydroxy-11-benzoyloxy-15-(tetrahydropyran-2-yloxy)prosta-5,13-dienoic acid methyl ester (prepared in accordance with the process described in European Patent Publication No. 98141, Reference Example 2) in 50 ml of dry methylene chloride, 1.12 ml of triethylamine and 0.58 ml of mesyl chloride were added at -20°C under an argon atmosphere. The resulting mixture was stirred for 15 minutes at the same temperature. The reaction mixture was poured into 150 ml of ice water. The mixture was extracted with diethyl ether. The extract was washed in succession with water and a saturated sodium chloride aqueous solution. After drying, the extract was concentrated under reduced pressure to give 3.23 g of the crude title compound having the following physical characteristics:

TLC (n-hexane : ethyl acetate = 2:1): Rf = 0.51;

NMR: δ = 8.1-7.7 (2H, m), 7.6-7.1 (3H, m), 5.7-4.9 (6H, m), 3.7 (3H, s), 3.0 (3H, s).

## Reference Example 2

Synthesis of:

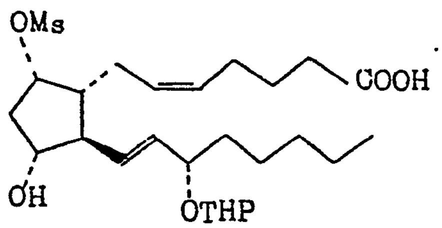

To a solution of 3.23 g of the 11-benzoyl compound (prepared in Reference Example 1) in 133 ml of methanol, a suspension of 12.6 g of lithium hydroxide monohydrate in 19 ml of water was added at 5°C. The mixture was stirred for 2 hours at the same temperature and for a further 5 hours at room temperature. The reaction mixture was poured into 300 ml of ice water and 1 N hydrochloric acid was added to the mixture to adjust to pH 3. Then the mixture was extracted with ethyl acetate. The extract was washed successively with water and a saturated sodium chloride aqueous solution. After drying, the extract was concentrated under reduced pressure. The residue was purified by column chromatography on silica gel

using a solvent mixture of n-hexane and ethyl acetate and then using ethyl acetate, as eluents to give 2.01 g of the title compound having the following physical characteristics:

TLC (chloroform : tetrahydrofuran : acetic acid = 30:6 :1): Rf = 0.44;

NMR: $\delta$ =6.4-5.6 (2H, m), 5.7-5.2 (4H, m), 5.15-4.9 (1H, m), 4.8-4.6 (1H, m), 4.3-3.1 (4H, m), 3.0 (3H, s);

IR: $\nu$ =3650-2400, 2930, 2860, 1720, 1340, 1170, 970 $cm^{-1}$.

Reference Example 3

Synthesis of:

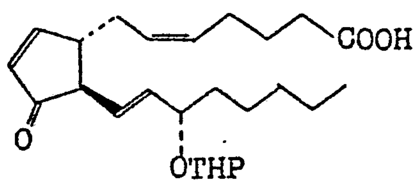

To a solution of 1.24 g of the 9-mesyl compound (prepared in Reference Example 2) in 12 ml of acetone, 4.5 ml of Jones' reagent was dropwise added at -30°C. The mixture was stirred for 20 minutes at -20°C. The reaction mixture was diluted with diethyl ether and then washed successively with water and a saturated sodium chloride aqueous solution, dried and concentrated under reduced pressure.

The residue was purified by column chromatography on silica gel using a solvent mixture of ethyl acetate and n-hexane as an eluent to give 610 mg of the title compound having the following physical characteristics:

TLC (ethyl acetate : n-hexane = 2 : 1): Rf = 0.27

NMR: $\delta$=7.64-7.52 (1H, m), 6.22-6.10 (1H, m), 5.7-5.2 (4H, m), 4.8-4.6 (1H, m), 1.0-0.8 (3H, m);

IR: $\nu$=1715, 1590, 1020 $cm^{-1}$.

The following compounds were obtained from compounds described in European Patent Publication No. 98141, Reference Examples 2(a), 2(b), 2(c) and 2(e), respectively, by the same procedures as described in Reference Examples 1, 2 and 3.

Table 1

| Reference Example No. | Structural Formula of Product | Physical Characteristic of Product: TLC (Rf) developing solvent in parenthesis | NMR (δ) | IR (ν) |
|---|---|---|---|---|
| 3 (a) | 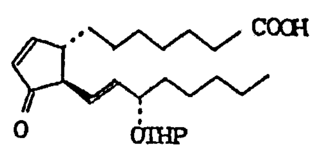 | Rf = 0.29 (ethyl acetate : n-hexane = 2 : 1) | 7.62~7.50 (1H, m), 6.20~6.10 (1H, m), 5.7~5.4 (2H, m), 4.8~4.6 (1H, m), 1.0~0.8 (3H, m) | 2940, 1735, 1715, 1590 |
| 3 (b) | 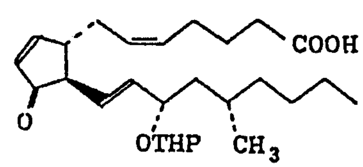 | Rf = 0.31 (ethyl acetate: n-hexane = 2 : 1) | 7.63~7.52 (1H, m), 6.21~6.10 (1H, m), 5.7~5.2 (4H, m), 4.8~4.6 (1H, m), 1.0~0.8 (6H, m) | 2940, 1710, 1590 |
| 3 (c) | 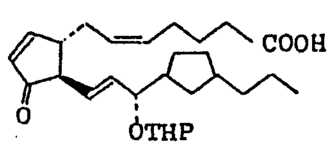 | Rf = 0.30 (ethyl acetate: n-hexane = 2 : 1) | 7.64~7.53 (1H, m), 6.23~6.10 (1H, m), 5.73~5.20 (4H, m), 4.8~4.6 (1H, m), 1.0~0.8 (3H, m) | 1713, 1590 |
| 3 (d) | 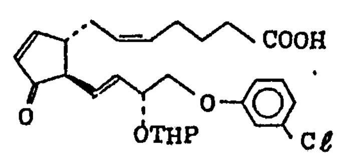 | Rf = 0.27 (ethyl acetate: n-hexane = 2 : 1) | 8.1~7.8 (2H, m), 7.6~6.7 (5H, m), 6.2~6.1 (1H, m), 5.7~5.2 (4H, m) | 1715, 1600, 1590 |

Reference Example 4

Synthesis of:

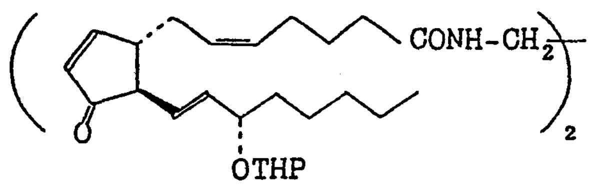

To a solution of 250 mg of the 15-THP compound (prepared in Reference Example 3) in 15 ml of methylene chloride, 0.125 ml of triethylamine and 78 μl of isobutyl chloroformate were dropwise added at room temperature under a nitrogen atmosphere. The resulting mixture was stirred for 15 minutes at the same temperature. Thereafter 20 μl of ethylenediamine was added to the reaction mixture followed by stirring for 15 minutes at room temperature. The reaction mixture was diluted with methylene chloride. The diluted mixture was washed in succession with a saturated sodium bicarbonate aqueous solution and a saturated sodium chloride aqueous solution, dried and concentrated under reduced pressure. The residue was purified by column chromatography on silica gel using a solvent mixture of n-hexane and ethyl acetate and then ethyl

acetate alone, as eluents to give 203 mg of the title compound having the following physical characteristics:

TLC (chloroform : THF : acetic acid = 10 : 2 : 1): Rf= 0.47;

NMR: δ= 7.57 (2H, dd), 6.2~6.1 (2H, m), 5.7~5.3 (8H, m), 4.76~4.6 (2H, m), 4.2~3.97 (2H, m), 3.97~3.78 (2H, m), 3.56~3.3 (6H, m), 2.94 ~2.77 (2H, m), 2.7~2.6 (2H, m), 1.0~0.8 (6H, m);

IR: ν = 3300, 1710, 1650, 1540, 1240, 1020, 980 $cm^{-1}$.

The following compounds were obtained in a manner similar to Reference Example 4. Triethylenediamine was used instead of ethylenediamine in Reference Example 4(e); tetramethylenediamine was used in Reference Example 4(f); 2-aminoethanol was used in Reference Example 4(g).

Table 2 (1)

| Reference Example No. | Starting Material | Structural Formula of Product | Physical Characteristic of Product: TLC (Rf) developing solvent | NMR (δ) | IR (ν) |
|---|---|---|---|---|---|
| 4(a) | Reference Example 3 (a) | 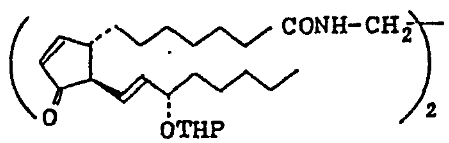 | Rf = 0.49 (chloroform: THF : AcOH =10 : 2 ; 1) | 7.56 (2H, dd), 6.2~6.1 (2H, m), 5.6~5.4 (4H, m), 4.7 (2H, m), 3.38 (4H, m) | 3300, 2940, 1710, 1650 |
| 4(b) | Reference Example 3 (b) | 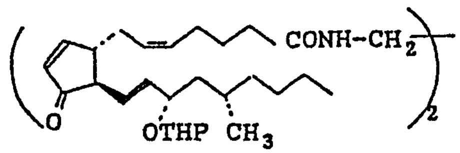 | Rf = 0.52 (chloroform : THF : AcOH =10 : 2 : 1) | 7.57 (2H, dd), 6.2~6.1 (2H, m), 5.7~5.3 (8H, m), 4.7 (2H, m), 3.38 (4H, m), 1.0~0.8 (12H, m) | 3300, 1710, 1650 |
| 4(c) | Reference Example 3 (c) | 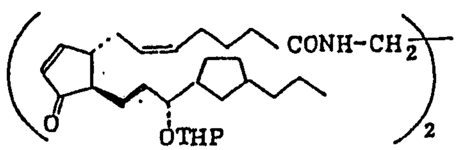 | Rf = 0.50 (chloroform: THF : AcOH =10 : 2 : 1) | 7.58 (2H, dd), 6.2~6.1 (2H, m), 5.7~5.3 (8H, m), 4.77~4.60 (2H, m), 3.6~3.3 (6H, m), 1.0~0.8 (6H, m) | 3300, 1712, 1650 |
| 4(d) | Reference Example 3 (d) | 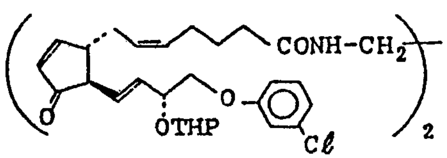 | Rf = 0.45 (chloroform: THF : AcOH =10 : 2 : 1) | 7.62 (2H, dd), 7.3~6.7 (4H, m), 6.2 (2H, m), 5.7~5.3 (8H, m), 4.7 (2H, m) | 3300, 1713, 1650, 1600 |
| 4(e) | Reference Example 3 | 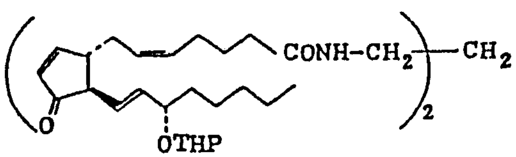 | Rf = 0.53 (chloroform: THF : AcOH =10 : 2 ; 1) | 7.56 (2H, dd), 6.4~6.2 (2H, broad s), 6.2~6.1 (2H, m), 5.8~5.3 (8H, m), 4.8~4.6 (2H, m), 1.0~0.8 (6H, m) | 3300, 1710, 1650 |

Table 2(2)

| Reference Example No. | Starting material | Structural Formula of Product | Physical Characteristic of Product | | |
|---|---|---|---|---|---|
| | | | TLC (Rf) developing solvent | NMR (δ) | IR (ν) |
| 4 (f) | Reference Example 3 | 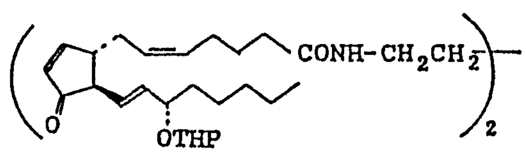 | Rf = 0.53 (chloroform : THF : AcOH =10 : 2 : 1) | 7.56 (2H, dd), 6.2~6.06 (2H, m), 6.05~5.8 (2H, broad s) 5.9~5.25 (8H, m), 4.75~4.55 (2H, m), 1.0~0.8 (6H, m) | 3300, 1710, 1650 |
| 4 (g) | Reference Example 3 | 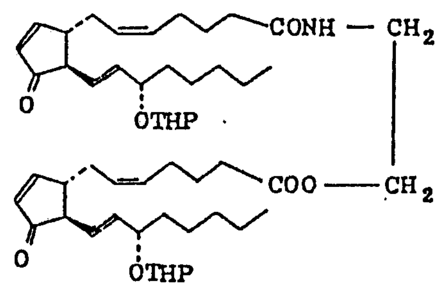 | Rf = 0.58 (chloroform : THF : AcOH =10 : 2 : 1) | 7.6~7.5 (2H, m), 6.2~6.08 (2H, m), 5.7~5.2 (8H, m), 4.74~4.6 (2H, m); 4.20~3.95 (4H, m), 1.0~0.8 (6H, m) | 3330, 1730, 1705, 1650, 1240, 1020, 975 |

Reference Example 5

Synthesis of:

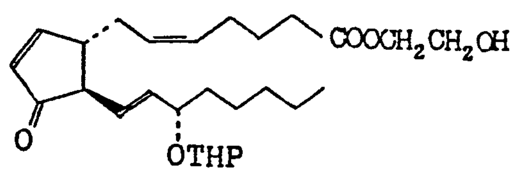

To a solution of 250 mg of the 15-THP compound (prepared in Reference Example 3) in 15 ml of methylene chloride, 0.125 ml of triethylamine and 0.1 ml of isobutyl chloroformate were dropwise added at room temperature under a nitrogen atmosphere. The resulting mixture was stirred for 15 minutes at the same temperature. Thereafter 0.334 ml of ethylene glycol and 0.485 ml of pyridine were added to the reaction mixture at room temperature followed by stirring for 30 minutes. The reaction mixture was diluted with methylene chloride. The diluted mixture was washed in succession with a saturated sodium bicarbonate aqueous solution and a saturated sodium chloride aqueous solution, dried and concentrated under reduced pressure. The residue was purified

by column chromatography on silica gel using a solvent mixture of n-hexane and ethyl acetate, as an eluent to give 179 mg of the title compound having the following physical characteristics:

TLC (chloroform : THF : acetic acid = 10 : 2 : 1): Rf = 0.6;

NMR: δ = 7.56 (1H, dd), 6.2~6.1 (1H, m), 5.7~5.3 (4H, m), 4.75~4.6 (1H, m), 4.30~4.15 (2H, m), 4.15~3.95 (1H, m), 3.95~3.7 (3H, m), 3.6~3.4 (1H, m), 2.92~2.75 (1H, m), 2.72~2.6 (1H, m), 1.0~0.8 (3H, m);

IR: ν = 3475, 1730, 1705, 1585, 1450, 1240, 1150, 1020, 980 $cm^{-1}$.

## Reference Example 6

Synthesis of:

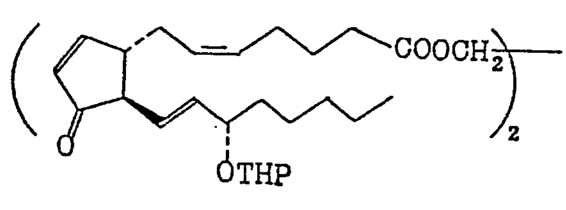

To a solution of 250 mg of the 15-THP compound (prepared in Reference Example 3) in 15 ml of methylene chloride, 0.1 ml of triethylamine and 78 μl of isobutyl chloroformate were dropwise added at room temperature under a nitrogen atmosphere. The resulting mixture was stirred for 15 minutes at the same temperature. Thereafter a solution of 172 mg of the ethylene glycol ester compound (prepared in Reference Example 5) in 5 ml of methylene chloride and 48 μl of pyridine were added to the reaction mixture followed by stirring for 18 hours at room temperature. The reaction mixture was diluted with methylene chloride. The diluted mixture was washed in succession with a saturated sodium bicarbonate aqueous solution and a saturated sodium chloride aqueous solution, dried and concentrated under reduced pressure. The residue was purified by column chromatography on silica gel using a solvent mixture of n-hexane and ethyl acetate, as an eluent to give 70 mg of the crude title compound having the following physical characteristics:

TLC (n-hexane : ethyl acetate = 1 : 1): Rf = 0.49;

NMR: δ = 7.65~7.5 (2H, m), 6.2~6.05 (2H, m), 5.7~5.2 (8H, m), 4.8~4.6 (2H, m), 4.25 (4H, s), 4.15~3.3 (6H, m), 2.95~2.55 (4H, m), 1.0~0.7 (6H, m);

IR: ν = 1735, 1705, 1585, 1150, 1020, 980 $cm^{-1}$.

## Example 1

Synthesis of:

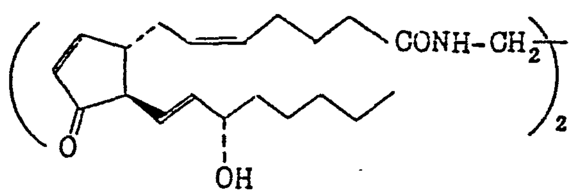

In 0.5 ml of THF, 283 mg of the dimer of the 15-THP compound (prepared in Reference Example 4) was dissolved and 5 ml of a 65% acetic acid aqueous solution was added to the resulting solution. The mixture was stirred at 80°C for 8 minutes. After allowing to cool, the reaction mixture was diluted with ethyl acetate. The mixture was washed in succession with an aqueous sodium bicarbonate solution and a saturated sodium chloride aqueous solution, dried and concentrated under reduced pressure. The residue was purified by column chromatography on silica gel using a solvent mixture of n-hexane and ethyl acetate, ethyl acetate alone and then a solvent mixture of ethyl acetate and methanol as eluents to give 183 mg of the title compound having the following physical characteristics.

TLC (chloroform : THF : acetic acid = 10 : 2 : 1): Rf = 0.06;

NMR: $\delta$ = 7.59 (2H, dd), 6.18 (2H, dd), 5.7~5.3 (8H, m), 4.2~4.0 (2H, m) 3.45~3.2 (4H, m), 2.95~2.8 (2H, m), 2.72~2.6 (2H, m), 1.0~0.8 (6H, m);

IR: $\nu$ = 3300, 1705, 1650, 1540, 1240, 1040, 975 $cm^{-1}$.

The following compounds were obtained in a manner similar to Example 1.

Table 3 (1)

| Example No. | Starting Material | Structural Formula of Product | Physical Characteristic of Product | | |
|---|---|---|---|---|---|
| | | | TLC (Rf) developing solvent | NMR (δ) | IR (ν) |
| 1 (a) | Reference Example 4 (a) | 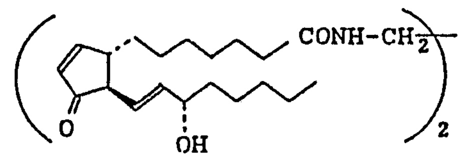 | Rf = 0.09 (chloroform: THF : AcOH =10 : 2 : 1) | 7.60 (2H, dd), 6.19 (2H, dd), 5.7~5.4 (4H, m), 4.2~4.0 (2H, m), 3.30 (4H, m), 0.89 (6H, t) | 3300, 1707, 1650 |
| 1 (b) | Reference Example 4 (b) | 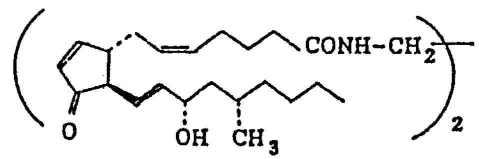 | Rf = 0.08 (chloroform: THF : AcOH =10 : 2 : 1) | 7.59 (2H, dd), 6.18 (2H, dd), 5.7~5.3 (8H, m), 3.40 (4H, m), 1.0~0.8 (12H, m) | 3300, 1705, 1650 |
| 1 (c) | Reference Example 4 (c) | 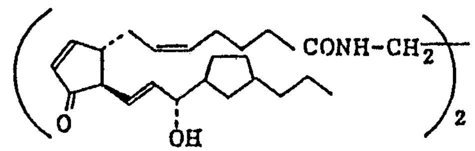 | Rf = 0.08 (chloroform: THF : AcOH =10 : 2 : 1) | 7.58 (2H, dd), 6.17 (2H, dd), 5.7~5.3 (8H, m), 3.32 (4H, m), 1.0~0.8 (6H, m) | 3300, 1706, 1650 |
| 1 (d) | Reference Example 4 (d) | 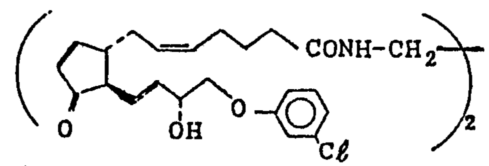 | Rf = 0.05 (chloroform: THF : AcOH =10 : 2 : 1) | 7.62 (2H, dd), 7.3~6.7 (4H, m), 6.2 (2H, dd), 5.7~5.3 (8H, m) | 3300, 1707, 1650, 1600 |
| 1 (e) | Reference Example 4 (e) | 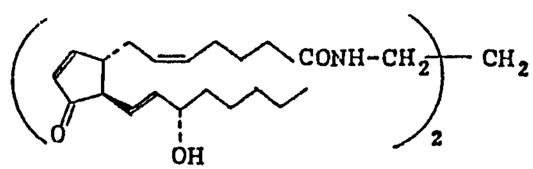 | Rf = 0.2 (chloroform: THF : AcOH =10 : 2 : 1) | 7.59 (2H, dd), 6.5~6.25 (2H, broad s), 6.16 (2H, dd), 5.7~5.3 (8H, m), 4.2~4.0 (2H, m), 3.45~3.15 (4H, m), 1.0~0.8 (6H, m) | 3300, 1710, 1650 |

Table 3 (2)

| Example No. | Starting Material | Structural Formula of Product | Physical Characteristic of Product | | |
|---|---|---|---|---|---|
| | | | TLC (Rf) developing solvent | NMR (δ) | IR (ν) |
| 1 (f) | Reference Example 4 (f) | 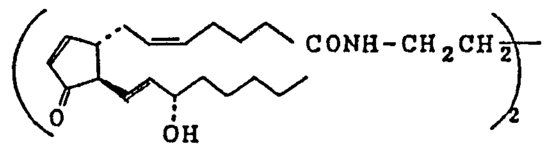 | Rf = 0.2 (chloroform: THF : AcOH =10 : 2 : 1) | 7.60 (2H, dd), 6.4~6.1 (4H, m), 5.8~5.25 (8H, m), 4.2~4.0 (2H, m), 3.45~3.15 (4H, m), 1.0~0.8 (6H, m) | 3300, 1705, 1650 |
| 1 (g) | Reference Example 4 (g) | 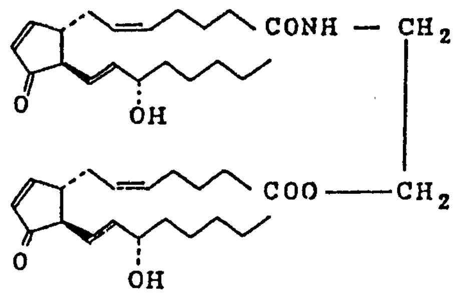 | Rf = 0.38 (chloroform: THF : AcOH =10 : 2 : 1) | 7.7~7.55 (2H, m), 6.24~6.12 (2H, m), 5.8~5.3 (8H, m), 4.2~4.0 (4H, m), 3.6~3.4 (2H, m), 3.0~2.78 (2H, m), 1.0~0.8 (6H, m) | 3350, 1700, 1650, 1580 |
| 1 (h) | Reference Example 6 | 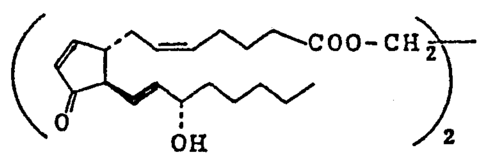 | Rf = 0.26 (chloroform: THF : AcOH =10 : 2 : 1) | 7.59 (2H, dd), 6.16 (2H, dd), 5.8~5.3 (8H, m), 4.27 (4H, s), 4.17~4.02 (2H, m), 2.95~2.78 (2H, m), 1.0~0.8 (6H, m) | 3450, 1735, 1705, 1590 |

Example 2

Synthesis of:

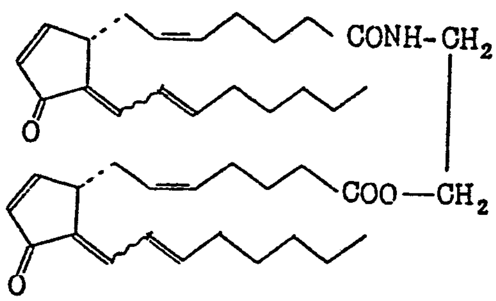

In 10 ml of THF, 116 mg of the dimer of the 9-deoxy-$\Delta^9$-$PGD_2$ (prepared in Example 1 (g)) was dissolved and 2.5 ml of 1N hydrochloric acid was added to the solution. The mixture was refluxed for 30 minutes. The reaction mixture was diluted with ethyl acetate. The mixture was washed in succession with an aqueous sodium bicarbonate solution and a saturated sodium chloride aqueous solution, dried and concentrated under reduced pressure. The residue was purified by column chromatography on silica gel using a solvent mixture of n-hexane and ethyl acetate as an eluent to give 20 mg of the title compound having the following physical characteristics.

TLC (chloroform : THF : acetic acid = 10 : 2 : 1): Rf = 0.6;

NMR: δ = 7.6~7.4 (2H, m), 7.0~6.88 (2H, m)
6.7~5.9 (6H, m), 5.7~5.2 (4H, m)
4.3~4.0 (2H, m), 3.9~3.2 (4H, m)
1.0~0.8 (6H, m);

IR: ν = 3350, 1730, 1690, 1650, 1370,
1240, 1040 $cm^{-1}$.

Example 3

Synthesis of:

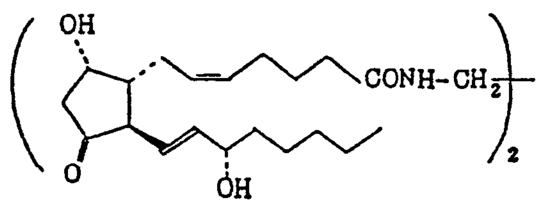

In 10 ml of methylene chloride, 140 mg of $PGD_2$ (prepared in accordance with the process described in British Patent No. 1403838, Example 1(B)) was dissolved and 66 µl of triethylamine and 52 µl of isobutyl chloroformate were dropwise added to the solution at room temperature under a nitrogen atmosphere. The mixture was stirred at the same temperature for 15 minutes. Then, 13.4 µl of ethylenediamine was added to the reaction mixture followed by stirring for 30

minutes at room temperature. The reaction mixture was diluted with methylene chloride. The mixture was washed in succession with an aqueous sodium bicarbonate solution and a saturated sodium chloride aqueous solution, dried and concentrated under reduced pressure to give white crystals. The obtained crystals were washed with diethyl ether to give 80 mg of the title compound having the following physical characteristics.

Melting Point: 75°C;

TLC (chloroform : methanol = 100 : 15): Rf = 0.43;

NMR: $\delta$= 6.9 (2H, broad s), 5.75~5.26 (8H, m), 4.42 (2H, broad s), 4.05 (2H, dd), 3.55~3.1 (4H, m), 2.81 (2H, dd), 1.0~0.8 (6H, m);

IR (KBr method): $\nu$= 3350, 1720, 1640, 1540, 1445, 1245, 970 $cm^{-1}$.

The following compound was obtained in a manner similar to Example 3, except that the crude product obtained by concentration under reduced pressure was purified by column chromatography on silica gel using methylene chloride and then a mixture of methylene chloride and methanol, as eluents.

(a)

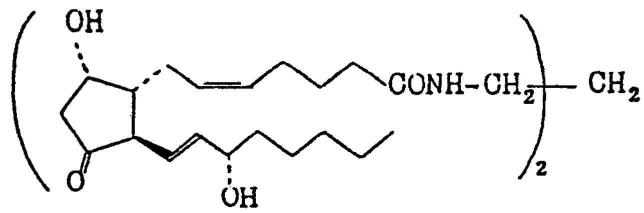

Starting Materials: $PGD_2$ and trimethylenediamine

TLC (chloroform : methanol = 10 : 1) : Rf = 0.21;

NMR: $\delta$ = 6.53 (2H, broad t), 5.73~5.33 (8H, m), 4.45 (2H, broad s), 4.08 (2H, dd), 3.4~3.1 (4H, m), 2.83 (2H, dd), 1.0~0.8 (6H, m);

IR: $\nu$ = 3350, 1730, 1640, 1530, 965 $cm^{-1}$.

## Example 4

Synthesis of:

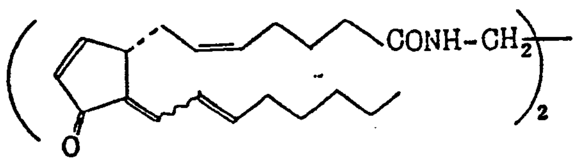

In 15 ml of methylene chloride, 200 mg of $PGD_2$ dimer (prepared in Example 3) was dissolved and, 0.2 ml of triethylamine was added to the solution at -10°C under a nitrogen atmosphere. Further 0.1 ml of mesyl chloride was dropwise added at the same temperature and the mixture was stirred for 15 minutes. Then, the temperature was gradually elevated to room temperature and stirring was conducted at room temperature for 2 hours. The reaction mixture was diluted with methylene chloride. The dilution was washed with a saturated sodium bicarbonate aqueous solution, dried and concentrated under reduced pressure. The residue was purified by column chromatography on silica gel using methylene chloride and then a solvent mixture of methylene chloride and methanol as an eluent to give 70 mg of the title compound having the following physical characteristics.

TLC (chloroform : methanol = 10 : 1): Rf = 0.54;

NMR: $\delta$ = 7.55~7.44 (2H, m), 6.94 (2H, broad d), 6.5~6.1 (6H, m), 5.6~5.25 (4H, m), 3.7~3.5 (2H, m), 3.5~3.3 (4H, m), 1.0~0.8 (6H, m);

IR: $\nu$ = 3300, 1690, 1650, 1630, 1540, 1450, 975 $cm^{-1}$.

The following compound was obtained in a manner similar to Example 4.

(a)

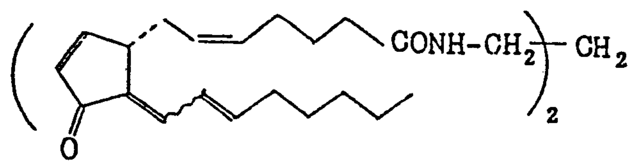

Starting materials: $PGD_2$ dimer prepared in Example 3 (a) and trimethylenediamine

TLC (chloroform : methanol = 10 : 1): Rf = 0.74;

NMR: δ = 7.55~7.42 (2H, m), 6.93 (2H, d), 6.5~6.0 (6H, m), 5.7~5.25 (4H, m), 3.7~3.5 (2H, m), 3.4~3.1 (4H, m), 1.0~0.8 (6H, m);

IR: ν = 3300, 1690, 1630, 1540, 1430 975 $cm^{-1}$.

The present invention includes within its scope pharmaceutical compositions which comprise at least one prostaglandin D derivative of general formula (I) or cyclodextrin clathrate thereof in association with a pharmaceutically acceptable carrier or coating.

In clinical practice the compounds of the present invention will normally be administered systemically or partially; usually by oral or parenteral (eg intravenous, subcutaneous or intramuscular) administration.

The dose to be administered is determined depending upon, for example, age, body weight, symptoms, the desired therapeutic effect, the route of administration, and the duration of the treatment.

In the human adult, the doses per person are generally between 5 mg and 500 mg by oral administration, and between 500 μg and 50 mg by parenteral, preferably intravenous administration, and can be administered up to several times per day, for the prevention or therapy (including therapy to secure remission) against leukemia, solid cancer, infection or leukocyto-penia, or to synchronize the cell cycle of tumour cells.

As mentioned above, the doses to be used depend on various factors. Therefore, there may be cases in which doses greater than the ranges specified above, or lower than the ranges specified above, may be used.

In the present invention, solid compositions for oral administration include compressed tablets, pills, dispersible powders and granules. In such solid compositions, one or more of the active compound (s) is, or are, admixed, with at least one inert diluent such as lactose, mannitol, glucose, hydroxypropyl cellulose, microcrystalline cellulose, starch, polyvinylpyrrolidone or magnesium metasilicate aluminate. The compositions may also comprise, as is normal practice, additional substances other than inert diluents, e.g. lubricating agents such as magnesium stearate, and disintegrating agents, such as cellulose calcium gluconate. The tablets or pills may, if desired, be made into enteric film-coated or gastric film-coated eg sugar-coated, gelatine-coated, hydroxypropylcellulose-coated or hydroxypropylmethylcellulose phthalate-coated tablets or pills; two or more layers may be used. The compositions for oral administration also include capsules of absorbable material such as gelatin.

Liquid compositions for oral administration include pharmaceutically acceptable emulsions, solutions, suspensions, syrups and elixirs containing inert diluents commonly used in the art such as distilled water or ethanol. Besides inert diluents such compositions may also comprise adjuvants such as wetting and suspending

agents, and sweetening, flavouring, perfuming and preserving agents.

Other compositions for oral administration include spray compositions which may be prepared by known methods and which comprise one or more active substances.

Preparations according to the invention for parenteral administration include sterile aqueous or non-aqueous solutions, suspensions or emulsions. Examples of aqueous solvents or suspending media are distilled water for injection and physiological salt solution. Examples of non-aqueous solvents or suspending media are propylene glycol, polyethylene glycol, vegetable oils such as olive oil, alcohols such as ethanol, and Polysorbate 80 (registered Trade Mark). These compositions may also include adjuvants such as preserving, wetting, emulsifying and dispersing agents. They may be sterilized, for example, by filtration through a bacteria-retaining filter, by incorporation of sterilizing agents in the compositions by irradiation. They may also be manufactured in the form of sterile solid compositions which can be dissolved in sterile water or some other sterile injectable medium immediately before use.

Other compositions include, for parenteral administration, liquids for external use, and endermic liniments such as ointments; suppositories for rectal

administration; and pessaries for vaginal administration.

The following Example illustrates pharmaceutical compositions according to the invention:

Example 5

A solution of 1 g of N,N'-bis[(5Z,13E)-(15S)-11-oxo-15-hydroxy-prosta-5,9,13-trienoyl]trimethylenediamine (prepared in Example 1 (e)) in 5 ml of ethanol was well mixed with 5 g of microcrystalline cellulose and dried sufficiently. To the resulting mixture, 100 mg of magnesium stearate, 20 mg of silicon dioxide, 10 mg of talc and 200 mg of cellulose calcium gluconate (CCG) were admixed and then microcrystalline cellulose was added to make the total weight 10 g. The resulting mixture was well mixed to make it homogeneous, and then tabletted in conventional manner to give 100 tablets each containing 10 mg of the active material.

0172962

CLAIMS

1. A prostaglandin D analogue of the formula:

$$(PG^1)-Y^1-(CH_2)_n-Y^2-(PG^2) \qquad (I)$$

[wherein $(PG^1)$ and $(PG^2)$, which may be the same or different, each represent a grouping of formula:

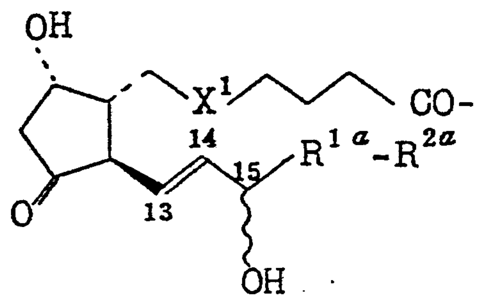

(II),

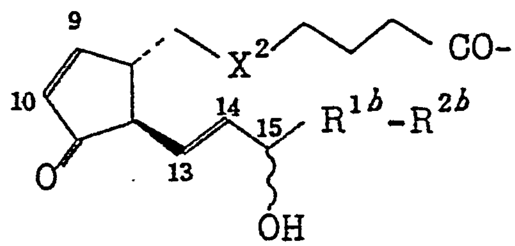

(III)

or

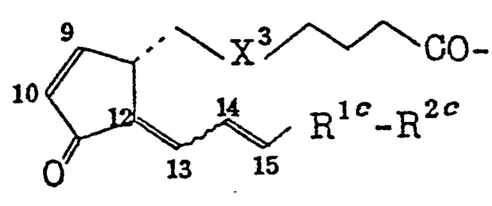

(IV)

(wherein $X^1$, $X^2$ and $X^3$, which may be the same or different, each represents an ethylene group or <u>cis</u>-vinylene group, $R^{1a}$, $R^{1b}$ and $R^{1c}$, which may be the same or different, each represents a single bond or a straight-chain or branched-chain alkylene group of 1 to 5 carbon atoms, $R^{2a}$, $R^{2b}$ and $R^{2c}$, which may be the same or different, each represents a straight-chain or branched-chain alkyl group of 1 to 8 carbon atoms or a cycloalkyl group of 4 to 7 carbon atoms either unsubstituted or substituted by at least one straight-chain or branched-chain alkyl group of 1 to 8 carbon atoms or a phenyl or phenoxy group either unsubstituted or substituted by at least one halogen atom, trifluoromethyl group or straight-chain or branched-chain alkyl group of 1 to 4 carbon atoms, the double bond between $C_9$-$C_{10}$ in the formulae (III) and (IV) is Z, the double bond between $C_{13}$-$C_{14}$ in the formulae (II) and (III) is E, and the double bonds between $C_{12}$-$C_{13}$ and between $C_{14}$-$C_{15}$ in the formula (IV), which may be in the same or different configuration are E, Z or a mixture thereof, with the proviso that when $R^{1a}$, $R^{1b}$ or $R^{1c}$ represent a single bond, $R^{2a}$, $R^{2b}$ or $R^{2c}$, respectively, do not represent a substituted or unsubstituted phenoxy group), both $Y^1$ and $Y^2$ represent an oxygen atom, or an imino group, or $Y^1$ represents an imino group and $Y^2$ represents an oxygen atom and n represents an integer of from 2 to 8], or a cyclodextrin clathrate thereof.

2. A prostaglandin D analogue according to claim 1 wherein in general formula (I) $(CH_2)_n$ represents a straight-chain alkylene group of 2 to 5 carbon atoms.

3. A prostaglandin D analogue according to claim 1 or 2 wherein in general formula (II), (III), or (IV), $R^{2a}$, $R^{2b}$ or $R^{2c}$, which may be the same or different, represents a butyl, pentyl or hexyl group either unsubstituted or substituted by one or two methyl or ethyl groups, a cyclobutyl, cyclopentyl or cyclohexyl group either unsubstituted or substituted by one methyl, ethyl, propyl or butyl group, or a phenyl or phenoxy group either unsubstituted or substituted by one chlorine atom, trifluoromethyl group or methyl or ethyl group.

4. A prostaglandin D analogue according to claim 1 or 2 wherein in general formula (II), (III) or (IV), $R^{1a} - R^{2a}$, $R^{1b} - R^{2b}$ or $R^{1c} - R^{2c}$, which may be the same or different, represents an n-pentyl, 1-methylpentyl, 1,1-dimethylpentyl, 2-methylhexyl, 1-butylcyclobutyl, cyclopentyl, 3-propylcyclopentyl, 4-butylcyclohexyl, benzyl, phenoxymethyl, 3-chlorophenoxymethyl or (3-trifluoromethyl) phenoxymethyl group.

5. A prostaglandin D analogue according to any one of claims 1 to 4 wherein $X^1$, $X^2$ and $X^3$ each represents a cis-vinylene group.

6. A prostaglandin D analogue according to any one of claims 1 to 5 wherein, in general formulae(II) and (III), the hydroxy group attached to the 15-position carbon atom is in α-configuration.

7. A prostaglandin D analogue according to claim 1 wherein in general formula (I) both $PG^1$ and $PG^2$ represent a group of the formula:

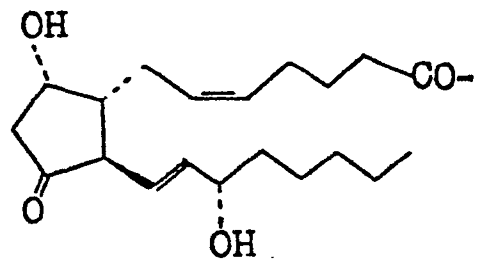

$I^1$

both $Y^1$ and $Y^2$ represent imino groups and $(CH_2)_n$ represents an ethylene group, both $PG^1$ and $PG^2$ represent the group of the formula $I^1$, both $Y^1$ and $Y^2$ represent imino groups and $(CH_2)_n$ represents a trimethylene group, both $PG^1$ and $PG^2$ represent a group of the formula:

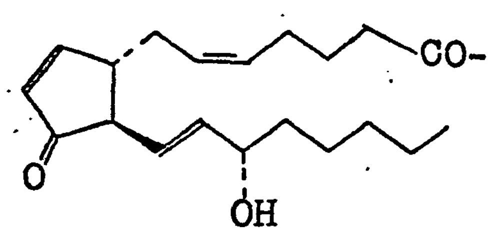

$I^2$

both $Y^1$ and $Y^2$ represent oxygen atoms and $(CH_2)_n$ represents an ethylene group, both $PG^1$ and $PG^2$ represent the group of the formula $I^2$, both $Y^1$ and $Y^2$ represent imino groups and $(CH_2)_n$ represent an ethylene group, both $PG^1$ and $PG^2$ represent the group of the formula $I^2$, both $Y^1$ and $Y^2$ represent imino groups and $(CH_2)_n$ represents a trimethylene group,

both $PG^1$ and $PG^2$ represent the group of the formula $I^2$, both $Y^1$ and $Y^2$ represent imino groups and $(CH_2)_n$ represents a tetramethylene group, both $PG^1$ and $PG^2$ represent the group of the formula $I^2$, $Y^1$ represents an imino group and $Y^2$ represents an oxygen atom, and $(CH_2)_n$ represents an ethylene group, both $PG^1$ and $PG^2$ represent a group of the formula:

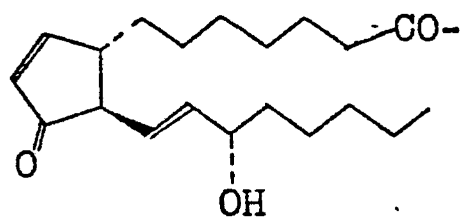

both $Y^1$ and $Y^2$ represent imino groups and $(CH_2)_n$ represents an ethylene group,
both $PG^1$ and $PG^2$ represent a group of the formula:

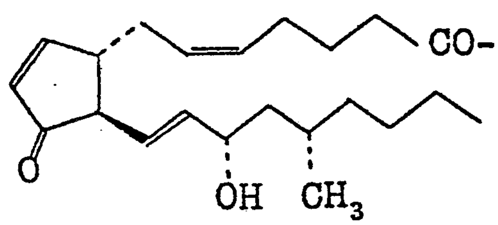

both $Y^1$ and $Y^2$ represent imino groups and $(CH_2)_n$ represents an ethylene group, both $PG^1$ and $PG^2$ represent a group of the formula:

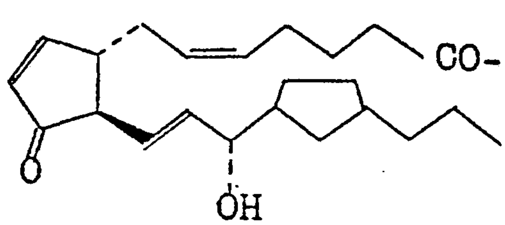

both $Y^1$ and $Y^2$ represent imino groups and $(CH_2)_n$ represents an ethylene group, both $PG^1$ and $PG^2$ represent a group of the formula:

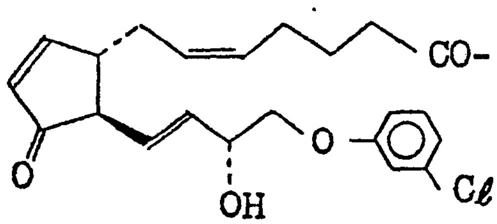

both $Y^1$ and $Y^2$ represent imino groups and $(CH_2)_n$ represents an ethylene group;

both $PG^1$ and $PG^2$ represent a group of the formula:

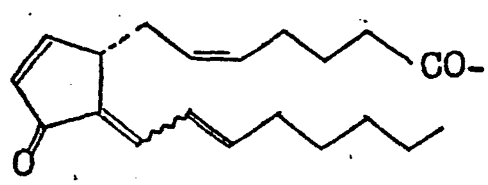

$I^3$

both $Y^1$ and $Y^2$ represent imino groups and $(CH_2)_n$ represents an ethylene group, both $PG^1$ and $PG^2$ represent the group of the formula $I^3$,

both $Y^1$ and $Y^2$ represent imino groups and $(CH_2)_n$ represents a trimethylene group, or both $PG^1$ and $PG^2$ represent the group of the formula $I^3$,

$Y^1$ represents an imino group and $Y^2$ represents an oxygen atom, and $(CH_2)_n$ represents an ethylene group.

8. A process for the preparation of prostaglandin D analogues of the formula:

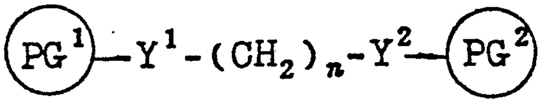

(I)

(wherein the various symbols are as defined in claim 1) which comprise

(A) the reaction of a compound of the formula:

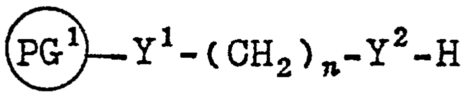

(IX)

(wherein the various symbols are as defined in claim 1) with a compound of the formula:

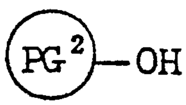

(X)

(wherein 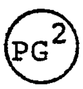 is as defined in claim 1);

(B) when the prostaglandin D analogues are of the formula:

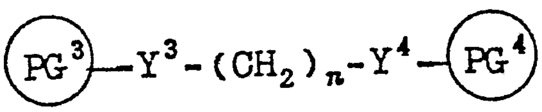

(IA)

[wherein 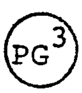 and 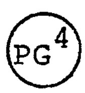, which may be the same or different, each represents a group of the formula:

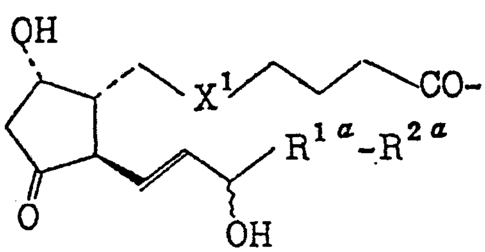

(II)

or

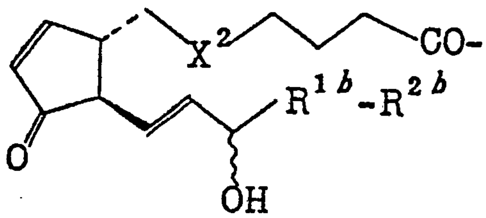

(III)

(wherein the various symbols are as defined in claim 1), $Y^3$ and $Y^4$ both represent an oxygen atom, or $Y^3$ represents an imino group and $Y^4$ represents an oxygen atom, and n is as defined in claim 1] the hydrolysis under acidic conditions of a compound of the formula:

$$PG^{3'}-Y^3-(CH_2)_n-Y^4-PG^{4'}$$ (XI)

[wherein $PG^{3'}$ and $PG^{4'}$, which may be the same or different, each represents a group of the general formula:

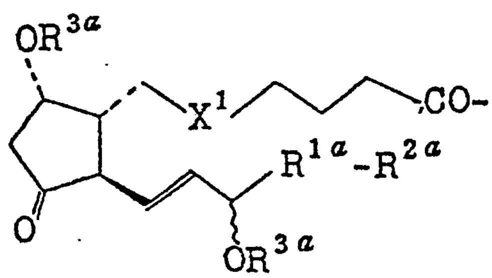

(XII)

or

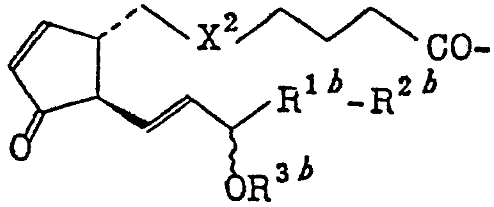

(XIII)

(wherein $R^{3a}$ and $R^{3b}$, which may be the same or different, each represents a tetrahydropyran-2-yl group or tetrahydrofuran-2-yl group either unsubstituted or substituted by at least one alkyl group, or a 1-ethoxyethyl group, and the other symbols are as defined in claim 1), and the other symbols are as defined in claim 1];

(C) when the prostaglandin D analogues are of the formula:

$$(PG^5)-Y^1-(CH_2)_n-Y^2-(PG^5) \quad (IB)$$

[wherein both groups $(PG^5)$, which are the same, represent a group of the general formula:

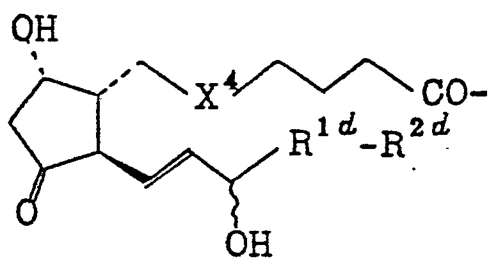

(IIa),

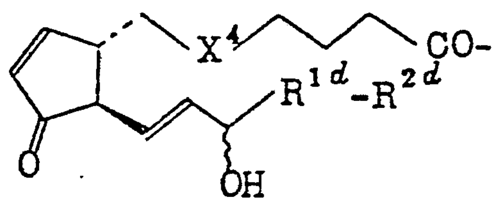

(IIIa)

or

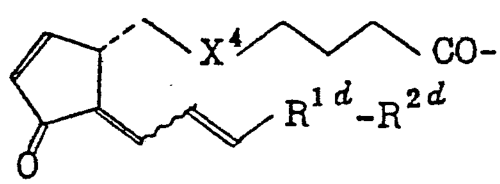

(IVa)

(wherein $X^4$ represents an ethylene group or a <u>cis</u>-vinylene group, $R^{1d}$ represents a single bond or a straight-chain or branched-chain alkylene group of 1 to 5 carbon atoms, $R^{2d}$ represents a straight-chain or branched-chain alkyl group of 1 to 8 carbon atoms, a cycloalkyl group of 4 to 7 carbon atoms either unsubstituted or substituted by at least one straight-chain or branched-chain alkyl group of 1 to 8 carbon atoms, or a phenyl or phenoxy group either unsubstituted or substituted by at least one halogen atom, trifluoromethyl group or straight-chain or branched-chain alkyl group of 1 to 4 carbon atoms, the double bond between $C_9$-$C_{10}$ in the general formulae (IIIa) and (IVa) is Z, the double bond between $C_{13}$-$C_{14}$ in the general formulae (IIa) and (IIIa) is E, the double bonds between $C_{12}$-$C_{13}$ and $C_{14}$-$C_{15}$ in the general formula (IVa), which may be in the same or different configurations are E, Z or EZ, with the proviso that when $R^{1d}$ represents a single bond, $R^{2d}$ does not represent a substituted or unsubstituted phenoxy group)
and the other symbols are as defined in claim 1]
the reaction of a compound of the general formula:

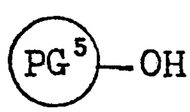

(XVI)

(wherein 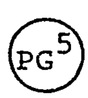 is as hereinbefore defined) with a compound of the general formula:

$HO-(CH_2)_n-OH$ (VI),

$H_2N-(CH_2)_n-OH$ (VII)

or

$H_2N-(CH_2)_n-NH_2$ (VIII)

(wherein n is as defined in claim 1);

(D) when the prostaglandin D analogues are of the formula:

$PG^6-Y^1-(CH_2)_n-Y^2-PG^6$ (IC)

[wherein both groups $PG^6$, which are the same, represent a group of the general formula:

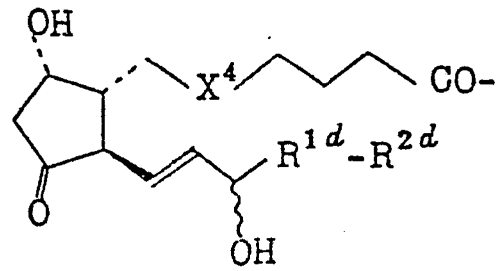

(IIa)

or

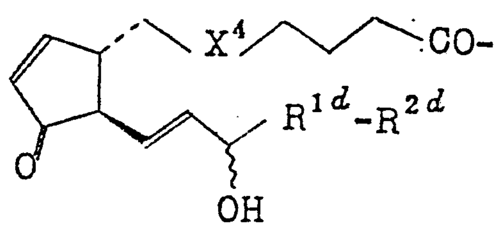

(IIIa)

(wherein the various symbols are as hereinbefore defined) and the other symbols are as defined in claim 1]

the hydrolysis under acidic conditions of a compound of the general formula:

$PG^{6'}-Y^1-(CH_2)_n-Y^2-PG^{6'}$ (XVII)

[wherein both groups $PG^6$, which are the same, represent a group of the formula:

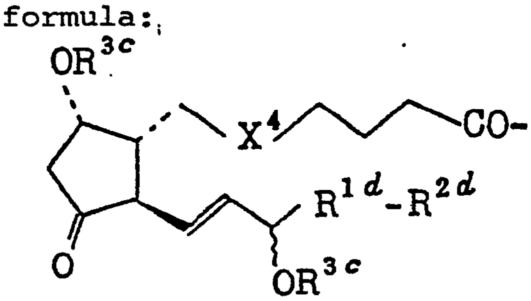

(XVIII)

or

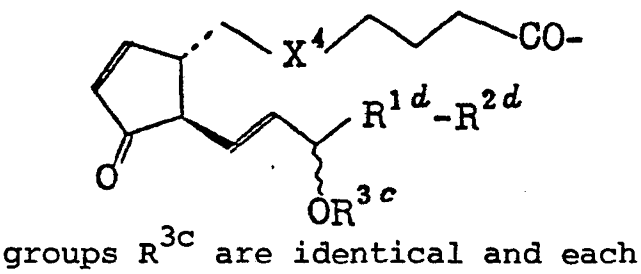

(XIX)

(wherein the groups $R^{3c}$ are identical and each represents a tetrahydropyran-2-yl group or tetrahydrofuran-2-yl group either unsubstituted or substituted by at least one alkyl group or a 1-ethoxyethyl group and the other symbols are as hereinbefore defined) and the other symbols are as defined in claim 1],

(E) when the prostaglandin D analogues are of the formula:

$$PG^7-Y^1-(CH_2)_n-Y^2-PG^8 \qquad (ID)$$

[wherein $PG^7$ and $PG^8$ each represents, a group represented by the general formula:

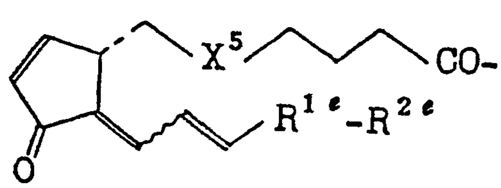

(XXI)

(wherein $X^5$ represents an ethylene group or a cis-vinylene group, $R^{1e}$ represents a single bond or a straight-chain or branched-chain alkylene group of 1 to 5 carbon atoms, $R^{2e}$ represents a straight-chain or branched-chain alkyl group of 1 to 8 carbon atoms, a cycloalkyl group of 4 to 7 carbon atoms either unsubstituted or substituted by at least one straight-chain or branched-chain alkyl group of 1 to 8 carbon atoms, or a phenyl or phenoxy group either unsubstituted or substituted by at least one halogen atom, trifluoromethyl group or straight-chain or branched-chain alkyl group of 1 to 4 carbon atoms, the double bond between $C_9$-$C_{10}$ is Z, and the double bonds between $C_{12}$-$C_{13}$ and $C_{14}$-$C_{15}$, which may be in the same or different configuration, are E, Z or EZ, with the proviso that when $R^{1e}$ represents a single bond, $R^{2e}$ does not represent a substituted or unsubstituted phenoxy group and pairs of $X^5$, $R^{1e}$ and $R^{2e}$ in $(PG^7)$ and $(PG^8)$ may be the same or different) and the other symbols are as defined in claim 1]

the reaction of a compound of the general formula:

$$(PG^3)-Y^1-(CH_2)_n-Y^2-(PG^4) \qquad (IF)$$

or

$$(PG^{3'})-Y^3-(CH_2)_n-Y^4-(PG^{4'}) \qquad (XI)$$

(wherein $Y^1$ and $Y^2$ are as defined in claim 1 and the other symbols are as hereinbefore defined) with an aqueous acid at the reflux temperature, or which comprises the reaction of a compound of the formula (IF) hereinbefore described with an alkylsulphonyl chloride or arylsulphonyl chloride, optionally followed by the step of converting a prostaglandin D analogue obtained into a cyclodextrin clathrate thereof.

9. A prostaglandin D analogue according to claim 1, or cyclodextrin clathrate thereof, when prepared by a process claimed in claim 8.

10. A pharmaceutical composition which comprises, as active ingredient, a prostaglandin D analogue of the formula (I) depicted in claim 1, wherein the various symbols are as defined in claim 1, or cyclodextrin clathrate thereof, in association with a pharmaceutical carrier of coating.

11. A prostaglandin D analogue as claimed in claim 1, or a cyclodextrin clathrate thereof for use in therapy.

12. A compound of formula (IX), (XI), or (XVII) wherein the various symbols are as defined in claims 1 and 8.

0172962



CLAIMS FOR AUSTRIA

1. A process for the preparation of a prostaglandin D analogue of the formula:

$$(PG^1)-Y^1-(CH_2)_n-Y^2-(PG^2) \qquad (I)$$

[wherein $(PG^1)$ and $(PG^2)$, which may be the same or different, each represent a grouping of formula:

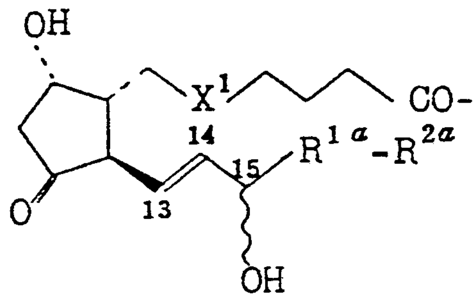

(II),

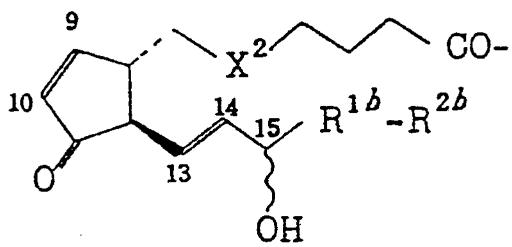

(III)

or

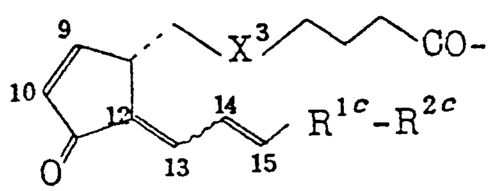

(IV)

(wherein $X^1$, $X^2$ and $X^3$, which may be the same or different, each represents an ethylene group or <u>cis</u>-vinylene group, $R^{1a}$, $R^{1b}$ and $R^{1c}$, which may be the same or different, each represents a single bond or a straight-chain or branched-chain alkylene group of 1 to 5 carbon atoms, $R^{2a}$, $R^{2b}$ and $R^{2c}$, which may be the same or different, each represents a straight-chain or branched-chain alkyl group of 1 to 8 carbon atoms or a cycloalkyl group of 4 to 7 carbon atoms either unsubstituted or substituted by at least one straight-chain or branched-chain alkyl group of 1 to 8 carbon atoms or a phenyl or phenoxy group either unsubstituted or substituted by at least one halogen atom, trifluoromethyl group or straight-chain or branched-chain alkyl group of 1 to 4 carbon atoms, the double bond between $C_9$-$C_{10}$ in the formulae (III) and (IV) is Z, the double bond between $C_{13}$-$C_{14}$ in the formulae (II) and (III) is E, and the double bonds between $C_{12}$-$C_{13}$ and between $C_{14}$-$C_{15}$ in the formula (IV), which may be in the same or different configuration are E, Z or a mixture thereof, with the proviso that when $R^{1a}$, $R^{1b}$ or $R^{1c}$ represent a single bond, $R^{2a}$, $R^{2b}$ or $R^{2c}$, respectively, do not represent a substituted or unsubstituted phenoxy group), both $Y^1$ and $Y^2$ represent an oxygen atom, or an imino group, or $Y^1$ represents an imino group and $Y^2$ represents an oxygen atom and n represents an integer of from 2 to 8], or a cyclodextrin clathrate thereof, characterised in that it is prepared by a process which comprises:

(A) the reaction of a compound of the formula:

$$(PG^1)-Y^1-(CH_2)_n-Y^2-H \qquad (IX)$$

(wherein the various symbols are as hereinbefore defined) with a compound of the formula:

$$(PG^2)-OH \qquad (X)$$

(wherein $(PG^2)$ is as hereinbefore defined);

(B) when the prostaglandin D analogue is of the formula:

$$(PG^3)-Y^3-(CH_2)_n-Y^4-(PG^4) \qquad (IA)$$

[wherein $(PG^3)$ and $(PG^4)$, which may be the same or different, each represents a group of the formula:

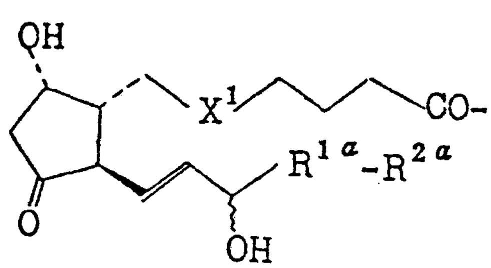

(II)

or

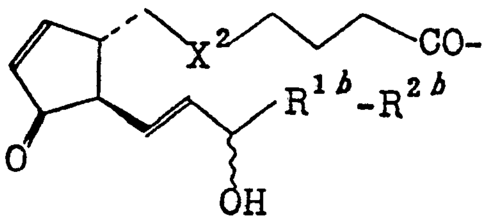

(III)

(wherein the various symbols are as hereinbefore defined), $Y^3$ and $Y^4$ both represent an oxygen atom, or $Y^3$ represents an imino group and $Y^4$ represents an oxygen atom, and n is as hereinbefore defined_7 the hydrolysis under acidic conditions of a compound of the formula:

$$(PG^{3'})-Y^3-(CH_2)_n-Y^4-(PG^{4'}) \qquad (X)$$

[wherein $(PG^{3'})$ and $(PG^{4'})$, which may be the same or different, each represents a group of the general formula:

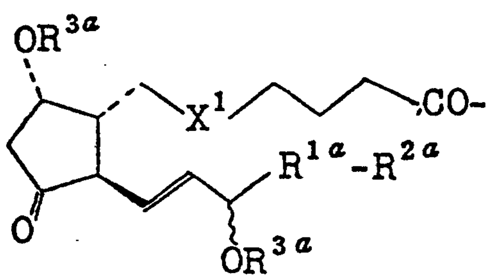

(II)

or

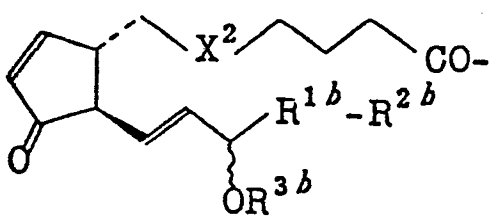

(XIII)

(wherein $R^{3a}$ and $R^{3b}$, which may be the same or different, each represents a tetrahydropyran-2-yl group or tetrahydrofuran-2-yl group either unsubstituted or substituted by at least one alkyl group, or a 1-ethoxyethyl group, and the other symbols are as hereinbefore defined), and the other symbols are as hereinbefore defined_7;

(C) when the prostaglandin D analogue is of the formula:

$$(PG^5)-Y^1-(CH_2)_n-Y^2-(PG^5) \quad (IB)$$

[wherein both groups $(PG^5)$, which are the same, represent a group of the general formula:

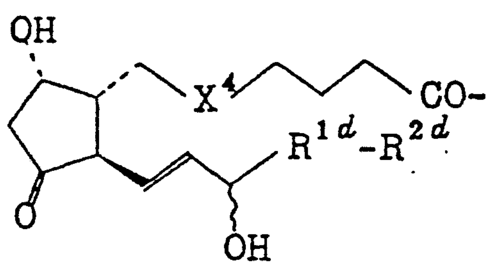

(IIa),

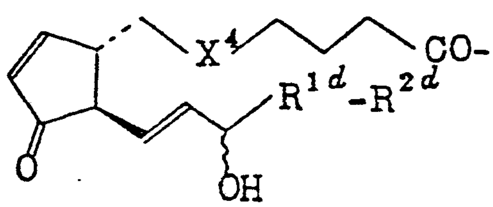

(IIIa)

or

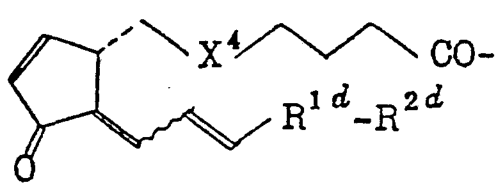

(IVa)

(wherein $X^4$ represents an ethylene group or a cis-vinylene group, $R^{1d}$ represents a single bond or a straight-chain or branched-chain alkylene group of 1 to 5 carbon atoms, $R^{2d}$ represents a straight-chain or branched-chain alkyl group of 1 to 8 carbon atoms, a cycloalkyl group of 4 to 7 carbon atoms either unsubstituted or substituted by at least one straight-chain or branched-chain alkyl group of 1 to 8 carbon atoms, or a phenyl or phenoxy group either unsubstituted or substituted by at least one halogen atom, trifluoromethyl group or straight-chain or branched-chain alkyl group of 1 to 4 carbon atoms, the double bond between $C_9$-$C_{10}$ in the general formulae (IIIa) and (IVa) is Z, the double bond between $C_{13}$-$C_{14}$ in the general formulae (IIa) and (IIIa) is E, the double bonds between $C_{12}$-$C_{13}$ and $C_{14}$-$C_{15}$ in the general formula (IVa), which may be in the same or different configurations are E, Z or EZ, with the proviso that when $R^{1d}$ represents a single bond, $R^{2d}$ does not represent a substituted or unsubstituted phenoxy group)

and the other symbols are as hereinbefore defined_7

the reaction of a compound of the general formula:

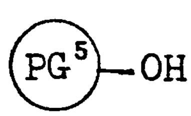 (XVI)

(wherein $PG^5$ is as hereinbefore defined) with a compound of the general formula:

$$HO-(CH_2)_n-OH \qquad (VI),$$

$$H_2N-(CH_2)_n-OH \qquad (VII)$$

or

$$H_2N-(CH_2)_n-NH_2 \qquad (VIII)$$

(wherein n is as hereinbefore defined);

(D) when the prostaglandin D analogue is of the formula:

$$(PG^6)-Y^1-(CH_2)_n-Y^2-(PG^6) \qquad (IC)$$

[wherein both groups $(PG^6)$, which are the same, represent a group of the general formula:

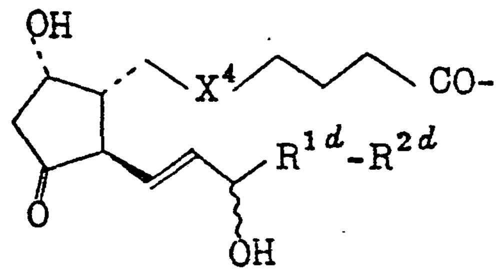

(IIa)

or

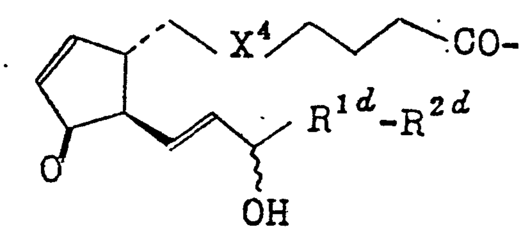

(IIIa)

(wherein the various symbols are as hereinbefore defined)]

the hydrolysis under acidic conditions of a compound of the general formula:

$$(PG^{6'})-Y^1-(CH_2)_n-Y^2-(PG^{6'}) \qquad (XVII)$$

[wherein both groups $PG^6$ , which are the same, represent a group of the formula:

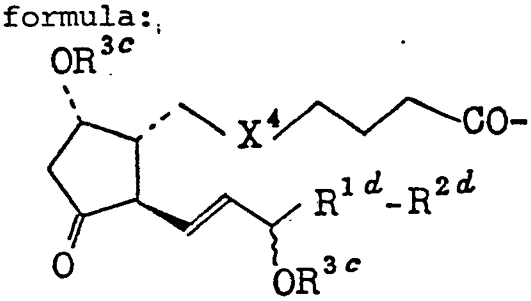

(XVIII)

or

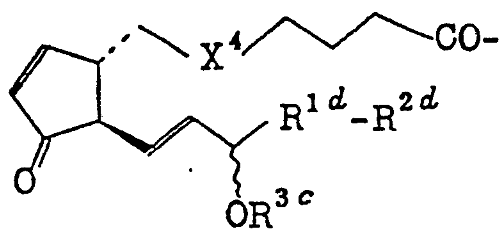

(XIX)

(wherein the groups $R^{3c}$ are identical and each represents a tetrahydropyran-2-yl group or tetrahydrofuran-2-yl group either unsubstituted or substituted by at least one alkyl group or a 1-ethoxyethyl group and the other symbols are as hereinbefore defined) 7;

(E) when the prostaglandin D analogue is of the formula:

$$PG^7 - Y^1 - (CH_2)_n - Y^2 - PG^8 \qquad (ID)$$

[wherein $PG^7$ and $PG^8$ each represents, a group represented by the general formula:

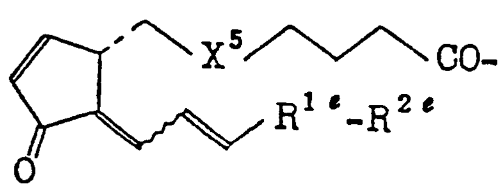

(XXI)

(wherein $X^5$ represents an ethylene group or a cis-vinylene group, $R^{1e}$ represents a single bond or a straight-chain or branched-chain alkylene group of 1 to 5 carbon atoms, $R^{2e}$ represents a straight-chain or branched-chain alkyl group of 1 to 8 carbon atoms, a cycloalkyl group of 4 to 7 carbon atoms either unsubstituted or substituted by at least one straight-chain or branched-chain alkyl group of 1 to 8 carbon atoms, or a phenyl or phenoxy group either unsubstituted or substituted by at least one halogen atom, trifluoromethyl group or straight-chain or branched-chain alkyl group of 1 to 4 carbon atoms, the double bond between $C_9$-$C_{10}$ is Z, and the double bonds between $C_{12}$-$C_{13}$ and $C_{14}$-$C_{15}$, which may be in the same or different configuration, are E, Z or EZ, with the proviso that when $R^{1e}$ represents a single bond, $R^{2e}$ does not represent a substituted or unsubstituted phenoxy group and pairs of $X^5$, $R^{1e}$ and $R^{2e}$ in $PG^7$ and $PG^8$ may be the same or different) and the other symbols are as hereinbefore defined_7;

the reaction of a compound of the general formula

$$PG^3-Y^1-(CH_2)_n-Y^2-PG^4 \qquad (IF)$$

or

$$PG^{3'}-Y^3-(CH_2)_n-Y^4-PG^{4'} \qquad (XI)$$

(wherein the various symbols are as hereinbefore defined) with an aqueous acid at the reflux temperature, or which comprises the reaction of a compound of the formula (IF) hereinbefore described with an alkylsulphonyl chloride or arylsulphonyl chloride,

optionally followed by the step of converting a prostaglandin D analogue obtained into a cyclodextrin clathrate thereof.

2. A process according to claim 1 characterised in that $R^{3a}$, $R^{3b}$ and $R^{3c}$ represent the tetrahydropyran-2-yl group.

3. A process according to claim 1 (A) characterised in that the compound of general formula (X) is reacted with isobutyl chloroformate or trimethylacetyl chloride in an inert organic solvent in the presence of a base at a temperature of from $0^{\circ}$ C to room temperature, and then reacting the anhydrous acid mixture of the compound of general formula (X) thus obtained with the compound of general formula (IX).

4. A process according to claim 1 (B) or 1 (D) characterised in that the hydrolysis under acidic conditions is carried out using a mixture of dilute hydrochloric acid and tetrahydrofuran, a mixture of dilute hydrochloric acid and methanol, a mixture of acetic acid, water and tetrahydrofuran, a mixture of phosphoric acid, water and tetrahydrofuran or a mixture of p-toluenesulphonic acid and anhydrous methanol.

5. A process according to claim 1 (C) characterised in that the compound of general formula (XVI) is reacted with isobutyl chloroformate or trimethylacetyl chloride in an inert organic solvent in the presence of a base at a temperature of 0°C to room temperature and then reacting the anhydrous acid mixture of the compound of general formula (XVI) thus obtained with a compound of general formula (VI), (VII) or (VIII).

6. A process according to claim 1 (E) characterised in that the reaction of the compound of general formula (IF) or (XI) with an aqueous acid at the reflux temperature is carried out in tetrahydrofuran using 1N hydrochloric acid.

7. A process according to claim 1 (E) characterised in that the reaction of a compound of general formula (IF) with an alkylsulphonyl chloride or arylsulphonyl chloride is carried out in an organic solvent in the presence of a base at a temperature of from -30° C to 0° C.

European Patent Office

# EUROPEAN SEARCH REPORT

0172962

Application number

EP 84 30 5959

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 228 152 (P. FERRUTI) * Column 3, line 21 - column 4, line 14; claim 1 * | 1,8 | C 07 C 177/00<br>C 08 B 37/16<br>A 61 K 31/557 |
| A | FR-A-2 273 554 (THE WELLCOME FOUNDATION) * Page 1, lines 19-26; page 3, line 9 - page 6, line 25; claims 1,20 * | 1,8 | |
| | | | **TECHNICAL FIELDS SEARCHED (Int. Cl.4)** |
| | | | C 07 C 177/00 |

The present search report has been drawn up for all claims

| Place of search | Date of completion of the search | Examiner |
|---|---|---|
| THE HAGUE | 17-04-1985 | BERTE M.J. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82